# FASCICULE DE BREVET EUROPEEN

(11) **EP 2 773 675 B1**
(45) Date de publication et mention de la délivrance du brevet: **01.03.2023**
(21) Numéro de dépôt: 12797948.2
(22) Date de dépôt: 02.11.2012
(51) Int. Cl.: C08B 37/00, A61K 47/36, C08L 5/02, A61K 31/721, A61K 38/28

(54) **FORMULATION À ACTION RAPIDE D'INSULINE COMPRENANT UN OLIGOSACCHARIDE**
SCHNELL WIRKENDE INSULINFORMULIERUNG MIT EINEM OLIGOSACCHARID
QUICK-ACTING INSULIN FORMULATION INCLUDING AN OLIGOSACCHARIDE

(30) Priorité: 02.11.2011 US 201113287793
(43) Date de publication de la demande: 10.09.2014
(73) Titulaire: Adocia, 69003 Lyon (FR)
(72) Inventeur: SOULA, Olivier, F-69330 Meyzieu (FR); SOULA, Rémi, F-69330 Meyzieu (FR); SOULA, Gérard, F-69330 Meyzieu (FR)
(74) Mandataire: Tripoz, Inès
(86) Numéro de dépôt international: PCT/FR2012/052543
(87) Numéro de publication internationale: WO 2013/064787

(56) Documents cités:
- WO-A1-2010/122385
- WO-A2-2010/041138
- WO-A2-2011/098962
- FR-A1- 2 943 538
- US-A1- 2012 094 902

## Description

La présente invention concerne une formulation à action rapide d'insuline,

Depuis la production d'insuline par génie génétique, au début des années 80, les patients diabétiques bénéficient d'insuline humaine pour se traiter. Ce produit a grandement amélioré cette thérapie puisque les risques immunologiques liés à l'utilisation d'insuline non humaine en particulier de porc se trouvent éliminés. Cependant, l'insuline humaine injectée par voie sous-cutanée a un effet hypoglycémiant seulement après -60 minutes, ce qui implique que les patients diabétiques traités avec l'insuline humaine doivent procéder à l'injection 30 minutes avant le repas.

Un des problèmes à résoudre pour améliorer la santé et le confort des patients diabétiques est de mettre à leur disposition des formulations d'insuline qui permettent d'apporter une réponse hypoglycémiante plus rapide que celle de l'insuline humaine et si possible s'approchant de la réponse physiologique de la personne saine. La sécrétion d'insuline endogène chez l'individu sain est immédiatement déclenchée par l'augmentation de la glycémie. L'objectif est de réduire le plus possible le délai entre l'injection d'insuline et le début du repas.

Aujourd'hui, il est admis que la mise à disposition de telles formulations est utile pour que la prise en charge de la maladie soit la meilleure possible.

Le génie génétique a permis d'apporter une réponse avec le développement d'insulines analogues rapides. Ces insulines sont modifiées sur un ou deux acides aminés pour être plus rapidement absorbées dans le compartiment sanguin après une injection sous-cutanée. Ces insulines lispro (Humalog^{®}, Lilly), aspart (Novolog^{®},Novo) et glulisine (Apidra^{®}, SanofiAventis) sont des solutions stables d'insuline avec une réponse hypoglycémiante plus rapide que celle de l'insuline humaine. Dès lors, les patients traités avec ces insulines analogues rapides peuvent procéder à l'injection d'insuline seulement 15 minutes avant le repas.

Le principe des insulines analogues rapides est de former des hexamères à la concentration de 100 UI/mL pour assurer la stabilité de l'insuline dans le produit commercial tout en favorisant la dissociation très rapide de ces hexamères en monomères après injection sous-cutanée afin d'obtenir une action rapide.

L'insuline humaine telle que formulée sous sa forme commerciale, ne permet pas d'obtenir une réponse hypoglycémiante proche en terme de cinétique de la réponse physiologique générée par le début d'un repas (hausse de la glycémie), car à la concentration d'usage (100 UI/mL), en présence de zinc et d'autres excipients tels que le phénol ou le m-crésol, elle s'assemble sous forme d'hexamère alors qu'elle est active sous forme de monomère et de dimère. L'insuline humaine est préparée sous forme d'hexamères pour être stable près de 2 ans à 4°C car sous forme de monomères, elle a une très forte propension à s'agréger puis à fibriller ce qui lui fait perdre son activité. De plus sous cette forme agrégée, elle présente un risque immunologique pour le patient.

Les dissociations des hexamères en dimères et des dimères en monomères retardent son action de près de 20 minutes comparativement à une insuline analogue rapide (Brange J., et al, Advanced Drug Delivery Review, 35,1999, 307-335).

La cinétique de passage des insulines analogues dans le sang, ainsi que leur cinétique de réduction de la glycémie, ne sont pas optimales et il existe un réel besoin d'une formulation ayant un temps d'action encore plus court afin de s'approcher des cinétiques de sécrétion d'insuline endogène chez les personnes saines.

La société Biodel a proposé une solution à ce problème avec une formulation d'insuline humaine comprenant de l'EDTA et de l'acide citrique telle que décrite dans la demande de brevet US200839365. L'EDTA par sa capacité à complexer les atomes de zinc et l'acide citrique par ses interactions avec les zones cationiques présentes à la surface de l'insuline sont décrits comme déstabilisant la forme hexamérique de l'insuline et réduisant ainsi son temps d'action.

Cependant, une telle formulation présente notamment comme inconvénient de dissocier la forme hexamérique de l'insuline qui est la seule forme stable susceptible de répondre aux exigences de stabilité de la réglementation pharmaceutique.

On connaît également au nom de la demanderesse, la demande PCT WO2010/122385 qui décrit des formulations d'insuline humaine ou analogue et qui permet de résoudre les différents problèmes évoqués ci-dessus par l'addition d'un polysaccharide substitué comprenant des groupes carboxyles.

Cependant, les exigences entraînées par l'usage chronique et intensif voire l'usage pédiatrique de telles formulations conduisent l'homme de l'art à rechercher à utiliser des excipients dont la masse molaire et la taille soient les plus réduites possibles pour en faciliter l'élimination.

La recherche de réduction de la taille des polysaccharides a conduit l'homme de l'art à s'intéresser aux oligosaccharides mais ceux-ci en raison de leur taille réduite ne présentent pas les mêmes propriétés que les polysaccharides car il y a perte de l'effet polymère comme cela est démontré dans les exemples comparatifs de la partie expérimentale, voir notamment les essais de solubilisation de l'insuline au point isoélectrique et les essais d'interaction avec une protéine modèle telle que l'albumine.

En dépit de ces résultats décourageants, la demanderesse a réussi à mettre au point des formulations susceptibles d'accélerer l'insuline en utilisant des oligomères en combinaison avec un composé polyanionique.

De façon surprenante, outre le fait que l'addition de ce composé polyanionique permet d'améliorer les performances insuffisantes des oligosaccharides, elle permet d'obtenir des performances égales à celles obtenues avec un polysaccharide, et cela même lorsque l'oligosaccharide seul ou le composé polyanionique seul n'a aucun effet.

De plus comme dans le cas de l'utilisation de polysaccharides, la nature hexamérique de l'insuline n'est pas affectée, donc la stabilité des formulations n'est pas affectée.

La présente invention permet de résoudre les différents problèmes ci-dessus exposés puisqu'elle permet notamment de réaliser une formulation d'insuline, humaine ou analogue, capable après administration, d'accélerer le passage de l'insuline humaine ou de ses analogues dans le sang et de réduire plus rapidement la glycémie en comparaison des produits commerciaux d'insuline correspondants.

Elle permet également de réduire significativement le temps de début d'action d'une formulation d'insuline analogue rapide.

L'invention consiste en une composition, en solution aqueuse, comprenant de l'insuline et au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Dans un mode de réalisation, l'indice de polydispersité Ip est compris entre 1,2 et 1,8.

On entend par degré de polymérisation DP le nombre moyen d'unités répétitives (monomères) par chaîne de polymère. Il se calcule en divisant la masse molaire moyenne en nombre par la masse molaire moyenne du motif répété.

On entend par masse molaire moyenne en nombre (Mn) la moyenne arithmétique des masses de chacune des chaînes de polymère. Ainsi pour un nombre ni de chaînes i de masse molaire Mi, on a Mn = (ΣiniMi)/(Σini).

La masse molaire moyenne en poids (Mw) est obtenue par Mw = (ΣiniMi2)/(ΣiniMi), ni étant le nombre de chaînes de polymère i de masse molaire Mi.

Les polymères peuvent également être caractérisés par la distribution de longueurs de chaînes, aussi appelée indice de polydispersité (Ip), et est égal au Mw divisé par le Mn.

Dans un mode de réalisation, la composition comprend en outre un composé polyanionique.

Dans un mode de réalisation, l'insuline est l'insuline humaine.

On entend par insuline humaine une insuline obtenue par synthèse ou recombinaison dont la séquence peptidique est la séquence de l'insuline humaine, incluant les variations alléliques et les homologues.

Dans un mode de réalisation, l'insuline est une insuline analogue.

On entend par insuline analogue une insuline recombinante dont la séquence primaire contient au moins une modification par rapport à la séquence primaire de l'insuline humaine.

Dans un mode de réalisation l'insuline analogue est choisie dans le groupe constitué par l'insuline lispro (Humalog^{®}), l'insuline aspart (Novolog^{®}, Novorapid^{®}) et l'insuline glulisine (Apidra^{®}).

Dans un mode de réalisation, l'insuline analogue est l'insuline lispro (Humalog^{®}).

Dans un mode de réalisation, l'insuline analogue est l'insuline aspart (Novolog^{®}, Novorapid^{®}).

Dans un mode de réalisation, l'insuline analogue est l'insuline glulisine (Apidra^{®}).

L'invention concerne également une formulation pharmaceutique d'insuline comprenant une composition selon l'invention.

Dans un mode de réalisation, elle concerne une formulation pharmaceutique caractérisée en ce que la concentration en insuline est comprise entre 240 et 3000 µM (40 à 500 UI/mL).

Dans un mode de réalisation, elle concerne une formulation pharmaceutique caractérisée en ce que la concentration en insuline est comprise entre 600 et 1200 µM (100 à 200 UI/mL).

Dans un mode de réalisation, elle concerne une formulation pharmaceutique caractérisée en ce que la concentration en insuline est 600 µM (100 UI/mL).

Dans un mode de réalisation, elle concerne une formulation pharmaceutique caractérisée en ce que la concentration en insuline est 1200 µM (200 UI/mL),

Dans un mode de réalisation, l'invention concerne un complexe entre l'insuline humaine et un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Elle concerne également l'utilisation de ce complexe pour préparer des formulations d'insuline humaine permettant, après administration, d'accélerer le passage de l'insuline humaine dans le sang et de réduire plus rapidement la glycémie par rapport à une formulation exempte d'oligosaccharide seul ou en mélange avec un composé polyanionique.

Dans un mode de réalisation, l'invention concerne un complexe entre une insuline analogue et un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Elle concerne également l'utilisation de ce complexe pour préparer des formulations d'insuline analogue permettant, après administration, d'accélerer le passage de l'insuline analogue dans le sang et de réduire plus rapidement la glycémie par rapport à une formulation exempte d'oligosaccharide seul ou en mélange avec un composé polyanionique,

L'invention concerne l'utilisation d'au moins un oligosaccharide dont le degré de polymérisation moyen est compris entre 3 et 6 et l'indice de polydispersité Ip est compris entre 1,1 et 2,0, en mélange avec un composé polyanionique, pour préparer une formulation pharmaceutique d'insuline permettant, après administration, d'accélerer le passage de l'insuline dans le sang et de réduire plus rapidement la glycémie par rapport à une formulation exempte d'oligosaccharide seul ou en mélange avec un composé polyanionique.

Dans un mode de réalisation, l'invention concerne l'utilisation d'au moins un oligosaccharide dont le degré de polymérisation moyen est compris entre 3 et 6 et l'indice de polydispersité Ip est compris entre 1,1 et 2,0, en mélange avec un composé polyanionique, pour préparer une formulation d'insuline analogue permettant, après administration, d'accélérer le passage de l'insuline analogue dans le sang et de réduire plus rapidement la glycémie par rapport à une formulation exempte d'oligosaccharide seul ou en mélange avec un composé polyanionique.

Il est connu de l'homme de l'art que le délai d'action des insulines est dépendant de la concentration en insuline. Seules les valeurs de délai d'action des formulations à 100 UI/mL sont documentées.

Les formulations d'insuline humaine « regular » sur le marché à une concentration de 500 µM (100 UI/mL) ont un délai d'action compris entre 50 et 90 minutes et une fin d'action d'environ 360 à 420 minutes chez l'humain. Le temps pour atteindre la concentration maximale en insuline dans le sang est compris entre 90 et 180 minutes chez l'humain.

Les formulations d'insulines analogues rapides sur le marché à une concentration de 600 µM (100 UI/mL) ont un délai d'action compris entre 30 et 60 minutes et une fin d'action d'environ 240-300 minutes chez l'humain. Le temps pour atteindre la concentration maximale en insuline dans le sang est compris entre 50 et 90 minutes chez l'humain.

L'invention concerne également une méthode de préparation d'une formulation d'insuline humaine ayant une concentration en insuline comprise entre 240 et 3000 µM (40 et 500 UI/mL), dont le délai d'action chez l'humain est inférieur à celui de la formulation de référence à la même concentration en insuline en l'absence d'oligosaccharide caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Dans un mode de réalisation ladite méthode comprend en outre une étape d'addition à ladite formulation d'au moins un composé polyanionique.

L'invention concerne également une méthode de préparation d'une formulation d'insuline humaine ayant une concentration en insuline comprise entre 600 et 1200 µM (100 et 200 UI/mL), dont le délai d'action chez l'humain est inférieur à 60 minutes caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Dans un mode de réalisation ladite méthode comprend en outre une étape d'addition à ladite formulation d'au moins un composé polyanionique.

L'invention concerne également une méthode de préparation d'une formulationd'insuline humaine ayant une concentration en insuline de 600 µM (100 UI/mL), dont le délai d'action chez l'humain est inférieur à 60 minutes caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Dans un mode de réalisation ladite méthode comprend en outre une étape d'addition à ladite formulation d'au moins un composé polyanionique.

L'invention concerne également une méthode de préparation d'une formulation d'insuline humaine ayant une concentration en insuline de 1200 µM (200 UI/mL), dont le délai d'action chez l'humain est inférieur d'au moins 10% à celui de la formulation de l'insuline humaine en l'absence d'oligosaccharide caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Dans un mode de réalisation ladite méthode comprend en outre une étape d'addition à ladite formulation d'au moins un composé polyanionique.

L'invention consiste en la préparation d'une formulation d'insuline humaine dite rapide caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, comportant des groupes fonctionnels carboxyles partiellement substitués,

Dans un mode de réalisation ladite méthode comprend en outre une étape d'addition à ladite formulation d'au moins un composé polyanionique.

L'invention concerne également une méthode de préparation d'une formulation d'insuline humaine à une concentration de 600 µM (100 UI/mL) dont le délai d'action chez l'humain est inférieur à 60 minutes, de préférence inférieur à 45 minutes, et encore de préférence inférieur à 30 minutes caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Ladite méthode comprend en outre une étape d'addition à ladite formulation d'au moins un composé polyanionique.

L'invention concerne également une méthode de préparation d'une formulation d'insuline analogue ayant une concentration en insuline comprise entre 240 et 3000 µM (40 et 500 UI/mL), dont le délai d'action chez l'humain est inférieur à celui de la formulation de référence à la même concentration en insuline en l'absence d'oligosaccharide caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Dans un mode de réalisation ladite méthode comprend en outre une étape d'addition à ladite formulation d'au moins un composé polyanionique

L'invention concerne également une méthode de préparation d'une formulation d'insuline analogue ayant une concentration en insuline comprise entre 600 et 1200 µM (100 et 200 UI/mL), dont le délai d'action chez l'humain est inférieur à celui de la formulation de référence à la même concentration en insuline analogue en l'absence d'oligosaccharide, caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Ladite méthode comprend en outre une étape d'addition à ladite formulation d'au moins un composé polyanionique

L'invention concerne également une méthode de préparation d'une formulation d'insuline analogue ayant une concentration en insuline de 600 pmol/L (100 UI/mL), dont le délai d'action chez l'humain est inférieur à celui de la formulation de référence à la même concentration en insuline analogue en l'absence d'oligosaccharide caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

L'invention concerne également une méthode de préparation d'une formulation d'insuline analogue ayant une concentration en insuline de 1200 µM (200 UI/mL), dont le délai d'action chez l'humain est inférieur d'au moins 10% à celui de la formulation de l'insuline analogue en l'absence d'oligosaccharide caractérisée en ce qu'elle comprend une étape d'addition à ladite formulation d'au moins un oligosaccharide dont le degré de polymérisation moyen est compris entre 3 et 6 ledit oligosaccharide comportant des groupes fonctionnels carboxyles partiellement substitués, les groupes fonctionnels carboxyles non substitués étant salifiables.

Ladite méthode comprend en outre une étape d'addition à ladite formulation d'au moins un composé polyanionique.

L'invention concerne également une méthode de préparation d'une formulation d'insuline analogue ayant une concentration en insuline de 1200 pmol/L (200 UI/mL), dont le délai d'action chez l'humain est inférieur à 30 minutes caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Dans un mode de réalisation ladite méthode comprend en outre une étape d'addition à ladite formulation d'au moins un composé polyanionique

L'invention consiste en la préparation d'une formulation d'insuline analogue dite très rapide caractérisée en ce qu'elle comprend une étape d'addition à ladite formulation d'au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables.

Ladite méthode comprend en outre une étape d'addition à ladite formulation d'au moins un composé polyanionique.

Dans un mode de réalisation l'insuline analogue est choisie dans le groupe constitué par l'insuline lispro (Humalog^{®}), l'insuline aspart (Novolog^{®}, Novorapid^{®}) et l'insuline glulisine (Apidra^{®}).

Dans un mode de réalisation, l'insuline analogue est l'insuline lispro (Humalog^{®}).

Dans un mode de réalisation, l'insuline analogue est l'insuline aspart (Novolog^{®}, Novorapid^{®)}.

Dans un mode de réalisation, l'insuline analogue est l'insuline glulisine (Apidra^{®}).

Dans un mode de réalisation, l'oligosaccharide est un oligosaccharide fonctionnalisé dont le degré de polymérisation moyen est compris entre 3 et 6 et l'indice de polydispersité Ip est compris entre 1,1 et 2,0 et comportant des groupes fonctionnels carboxyles.

Ledit oligosaccharide est choisi parmi les oligosaccharides fonctionnalisés dont le degré de polymérisation est compris entre 3 et 6 et l'indice de polydispersité Ip est compris entre 1,1 et 2,0, constitués en majorité de liaisons glycosidiques de type (1,6).

Dans un mode de réalisation, l'oligosaccharide constitué en majorité de liaisons glycosidiques de type (1,6) est un dextrane fonctionnalisé comportant des groupes fonctionnels carboxyles.

Ledit oligosaccharide est fonctionnalisé par au moins un dérivé de la phénylalanine, noté Phe :
- ledit dérivé de la phénylalanine étant greffé ou lié à l'oligosaccharide par couplage avec une fonction acide, ladite fonction acide étant une fonction acide portée par un bras de liaison R lié à l'oligosaccharide par une fonction F, ladite fonction F résultant du couplage entre le bras de liaison R et une fonction -OH de l'oligosaccharide,
- F étant soit une fonction ester, carbamate ou éther,
- R étant une chaîne comprenant entre 1 et 15carbones, éventuellement branchée et/ou insaturée, comprenant un ou plusieurs hétéroatomes, tels que O, N ou/et S, et ayant au moins une fonction carboxyle,
- Phe étant un reste d'un dérivé de la phénylalanine, de configuration absolue L ou D, produit du couplage entre la fonction amine de la phénylalanine et au moins une fonction acide portée par le groupement R et/ou une fontion acide portée par l'oligosaccharide comportant des groupes fonctionnels carboxyles.

Dans un mode de réalisation, l'oligosaccharide fonctionnaliséest choisi parmi les oligosaccharides dont le degré de polymérisation moyen est compris entre 3 et 13 et l'indice de polydispersité Ip est supérieur à 1,0,de formule générale I suivante : 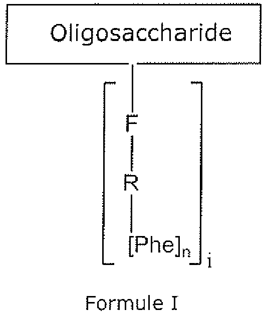
- l'oligosaccharide est un dextrane,
- F résulte du couplage entre le bras de liaison R et une fonction -OH de l'oligosaccharide et étant soit une fonction ester, carbamate ou éther,
- R est une chaîne comprenant entre 1 et 15 carbones, éventuellement branchée et/ou insaturée, comprenant un ou plusieurs hétéroatomes, tels que O, N ou/et S, et ayant au moins une fonction carboxyle,
- Phe est un reste d'un dérivé de phénylalanine, de configuration absolue L ou D, produit du couplage entre la fonction amine du dérivé de la phénylalanine et au moins une fonction acide portée par le groupement R avant rattachement à Phe,
- n représente la fraction molaire des R substitués par Phe et est comprise entre 0,1 et 0,9, de préférence entre 0,2 et 0,8, encore de préférence entre 0,3 et 0,7, encore de préférence entre 0,3 et 0,5 ;
- i représente la fraction molaire moyenne des groupements F-R-[Phe]ₙ portés par unité saccharidique et est comprise entre 0,5 et 3,0, de préférence entre 1,0 et 2,5, de préférence entre 1,2 et 2,2, de préférence entre 1,4 et 2,0 ;
- lorsque R n'est pas substitué par Phe, alors le ou les acides du groupement R sont des carboxylates de cation, alcalin de préférence comme Na⁺, K⁺, Ca²⁺ ou Mg²⁺.

Dans un mode de réalisation, n qui représente le degré de substitution des R substitués par Phe est comprise entre 0,1et 0,9, de préférence entre 0,2 et 0,8, de préférence entre 0,3et 0,7, encore de préférence entre 0,3et 0,5.

Dans un mode de réalisation, n qui représente la fraction molaire des R substitués par Phe est comprise entre 0,2 et 0,9, de préférence entre 0,3 et 0,8, de préférence entre 0,3 et 0,6, encore de préférence entre 0,3 et 0,5.

Dans un mode de réalisation, F est une fonction éther.

Dans un mode de réalisation, F est une fonction carbamate.

Dans un mode de réalisation, F est une fonction ester.

Dans un mode de réalisation R est une chaîne comprenant 1 carbone.

Dans un mode de réalisation, l'oligosaccharide selon l'invention est caractérisé en ce que le groupe R avant rattachement éventuel à Phe, est choisi dans les groupes suivants : 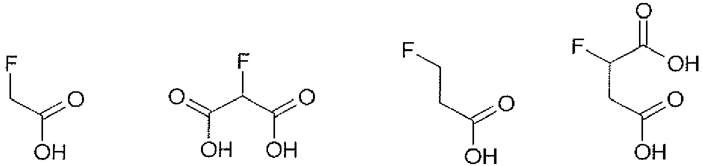 ou leurs sels de cations alcalins choisis dans le groupe constitué de Na⁺ ou K⁺,

Dans un mode de réalisation, l'oligosaccharide selon l'invention est caractérisé en ce que le groupe R avant rattachement éventuel à Phe, est issu de l'acide citrique.

Dans un mode de réalisation, l'oligosaccharide selon l'invention est caractérisé en ce que le groupe R avant rattachement éventuel à Phe, est issu de l'acide malique

Dans un mode de réalisation, l'oligosaccharide selon l'invention est caractérisé en ce que le dérivé de la phénylalanine est choisi dans le groupe constitué de la phénylalanine, l'alpha-methyl-phénylalanine, la 3,4 dihydroxyphénylalanine, la tyrosine, l'alpha-methyl-tyrosine, la O-methyl-tyrosine, l'alpha-phénylglycine, la 4-hydroxyphénylglycine, la 3,5-dihydroxyphénylglycine et leurs sels de cations alcalins et le phénylalaninol , le phénylalaninamide, l'éthyle benzyle amine, lesdits dérivés étant de configuration absolue L ou D.

Dans un mode de réalisation, l'oligosaccharide selon l'invention est caractérisé en ce que le dérivé de la phénylalanine est choisi dans le groupe constitué de la phénylalanineet ses sels de cations alcalins, le phénylalaninol, le phénylalaninamide, l'éthyle benzyle amine lesdits dérivés étant de configuration absolue L ou D.

Dans un mode de réalisation, l'oligosaccharide selon l'invention est caractérisé en ce que le dérivé de la phénylalanine est choisi dans le groupe constitué de la phénylalanine, l'alpha-methyl-phénylalanine, la 3,4 dihydroxyphénylalanine la tyrosine, l'alpha-methyl-tyrosine, la O-methyl-tyrosine, l'alpha-phénylglycine, la 4-hydroxyphénylglycine, la 3,5-dihydroxyphénylglycine et leurs sels de cations alcalins, lesdits dérivés étant de configuration absolue L ou D.

Dans un mode de réalisation, l'oligosaccharide selon l'invention est caractérisé en ce que le dérivé de la phénylalanine est la phénylalanine et ses sels de cations alcalins, ledit dérivé étant de configuration absolue L ou D.

Dans un mode de réalisation, l'oligosaccharide selon l'invention est caractérisé en ce que le dérivé de la phénylalanineest l'alpha-methyl-phénylalanine et ses sels de cations alcalins, ledit dérivé étant de configuration absolue L ou D.

Dans un mode de réalisation, l'oligosaccharide selon l'invention est caractérisé en ce que le dérivé de la phénylalanineest la 3,4 dihydroxyphénylalanine et ses sels de cations alcalins, ledit dérivé étant de configuration absolue L ou D.

Dans un mode de réalisation, l'oligosaccharide selon l'invention est caractérisé en ce que le dérivé de la phénylalanine est la tyrosine et ses sels de cations alcalins, ledit dérivé étant de configuration absolue L ou D.

Dans un mode de réalisation, l'oligosaccharide selon l'invention est caractérisé en ce que le dérivé de la phénylalanineest l'alpha-methyl-tyrosine et ses sels de cations alcalins, ledit dérivé étant de configuration absolue L ou D.

Dans un mode de réalisation, l'oligosaccharide selon l'invention est caractérisé en ce que le dérivé de la phénylalanineest la O-methyl-tyrosine et ses sels de cations alcalins, ledit dérivé étant de configuration absolue L ou D.

Dans un mode de réalisation, l'oligosaccharide selon l'invention est caractérisé en ce que le dérivé de la phénylalanineest l'alpha-phénylglycine et ses sels de cations alcalins, ledit dérivé étant de configuration absolue L ou D.

Dans un mode de réalisation, l'oligosaccharide selon l'invention est caractérisé en ce que le dérivé de la phénylalanineest la 4-hydroxyphénylglycine et ses sels de cations alcalins, ledit dérivé étant de configuration absolue L ou D.

Dans un mode de réalisation, l'oligosaccharide selon l'invention est caractérisé en ce que le dérivé de la phénylalanineest la 3,5-dihydroxyphénylglycineet ses sels de cations alcalins, ledit dérivé étant de configuration absolue L ou D.

Dans un mode de réalisation les dérivés de phénylalanine sont sous forme d'un mélange racémique.

Dans un mode de réalisation les dérivés de phénylalanine sont sous forme d'isomères de configuration absolue D, isolés.

Dans un mode de réalisation les dérivés de phénylalanine sont sous forme d'isomères de configuration absolue L, isolés.

Dans un mode de réalisation, l'oligosaccharide est choisi parmi les oligodextranes.

Dans un mode de réalisation, l'oligodextrane a une masse molaire moyenne en nombre inférieure à 3500 g/mol.

Dans un mode de réalisation, l'oligodextrane a une masse molaire moyenne en nombre inférieure à 2500 g/mol.

Selon l'invention, l'oligosaccharide a un degré de polymérisation moyen compris entre 3 et 6.

Selon l'invention, l'indice de polydispersité Ip est compris entre 1,1 et 2,0.

Dans un mode de réalisation, l'indice de polydispersité Ip est compris entre 1,2 et 1,8.

Dans un mode de réalisation, le composé polyanionique est une molécule anionique.

Selon l'invention, les molécules anioniques sont choisis dans le groupe constitué de l'acide citrique, l'acide aspartique, l'acide glutamique, l'acide malique, l'acide tartrique, l'acide succinique, l'acide adipique, l'acide oxalique, le triphosphate et leur sels de Na⁺, K⁺, Ca²⁺ ou Mg²⁺.

Dans un mode de réalisation, la molécule anionique est l'acide citrique et ses sels de Na⁺, K⁺, Ca²⁺ ou Mg²⁺.

Dans un mode de réalisation, le composé polyanionique est un polymère anionique.

Selon l'invention, les polymères anioniques sont choisis dans le groupe constitué de l'acide dextraneméthylcarboxylique, l'acide polyglutamique, l'acide polyaspartique, le PAA (acide polyacrylique), l'alginate, l'acide hyaluronique, les polymères à base d'acide glucuronique ou à base d'acide galacturonique et leur sels de Na⁺, K⁺, Ca²⁺ ou Mg²⁺.

Dans un mode de réalisation, le polymère anionique a une masse molaire moyenne en nombre comprise entre 1kg/mol et 15 kg/mol.

Dans un mode de réalisation, le polymère anionique a une masse molaire moyenne en nombre comprise entre 1 kg/mol et 10 kg/mol.

Dans un mode de réalisation, le polymère anionique a une masse molaire moyenne en nombre comprise entre 1 kg/mol et 8 kg/mol.

Dans un mode de réalisation, le polymère anionique est un dextrane synthétique porteur de fonctions carboxyles,

Dans un mode de réalisation, le dextrane synthétique porteur de fonctions carboxyles est choisi dans le groupe constitué des carboxyméthyldextrane, carboxyéthyldextrane, acide dextrane succinique, acide dextrane 2-butanedioique, acide dextrane propanedioique.

Dans un mode de réalisation, le polymère anionique est un carboxyméthyldextrane dont la fraction molaire en carboxyméthyl est comprise entre 0,5 et 3.

Dans un mode de réalisation, le polymère anionique est un carboxyméthyldextrane dont la fraction molaire en carboxyméthyl est comprise entre 0,5 et 2,5.

Dans un mode de réalisation, le polymère anionique est un carboxyméthyldextrane dont la fraction molaire en carboxyméthyl est comprise entre 1,0 et 2,0.

Dans un mode de réalisation, le polymère anionique est un acide dextrane succinique dont la fraction molaire en acide succinique est comprise entre 0,5 et 3.

Dans un mode de réalisation, le polymère anionique est un acide dextrane succinique dont la fraction molaire en acide succinique est comprise entre 0,5 et 2,5.

Dans un mode de réalisation, le polymère anionique est un acide dextrane succinique dont la fraction molaire en acide succinique est comprise entre 1,0 et 2,0.

Dans un mode de réalisation, le polymère anionique est un acide dextrane 2-butanedioique dont la fraction molaire en acide 2-butanedioique est comprise entre 0,2 et 3.

Dans un mode de réalisation, le polymère anionique est un acide dextrane 2-butanedioique dont la fraction molaire en acide 2-butanedioique est comprise entre 0,5 et 2,5.

Dans un mode de réalisation, le polymère anionique est un acide dextrane 2-butanedioique dont la fraction molaire en acide 2-butanedioique est comprise entre 0,7 et 2,0.

Dans un mode de réalisation, le polymère anionique est un acide dextrane propanedioique dont la fraction molaire en acide propanedioique est comprise entre 0,2 et 3.

Dans un mode de réalisation, le polymère anionique est un acide dextrane propanedioique dont la fraction molaire en acide propanedioique est comprise entre 0,5 et 2,5.

Dans un mode de réalisation, le polymère anionique est un acide dextrane propanedioique dont la fraction molaire en acide propanedioique est comprise entre 0,7 et 2,0.

Dans un mode de réalisation, le composé polyanionique est choisi parmi les composés anioniques constitués d'un squelette formé d'un nombre discret p compris entre 1 et 8 (1 ≤ p ≤ 8) d'unités saccharidiques identiques ou différentes, liées par des liaisons glycosidiques identiques ou différentes naturellement porteurs de groupe carboxyles ou substitués par des groupes carboxyles, et leurs sels.

Dans un mode de réalisation, le composé polyanionique constitué d'un squelette formé d'un nombre discret d'unités saccharidiques est obtenu à partir d'un composé disaccharide choisi dans le groupe constitué par le tréhalose, le maltose, le lactose, le saccharose, le cellobiose, l'isomaltose, le maltitol et l'isomaltitol.

Dans un mode de réalisation, le composé polyanionique constitué d'un squelette formé d'un nombre discret d'unités saccharidiques est obtenu à partir d'un composé constitué d'un squelette formé d'un nombre discret d'unités saccharidiques choisi dans le groupe constitué par le maltotriose, le maltotétraose, le maltopentaose, le maltohexaose, le maltoheptaose, le maltooactose et l'isomaltotriose

Dans un mode de réalisation, le composé polyanionique constitué d'un squelette formé d'un nombre discret d'unités saccharidiques est choisi dans le groupe constitué par le carboxyméthylmaltotriose, le carboxyméthylmaltotétraose, le carboxyméthylmaltopentaose, le carboxyméthylmaltohexaose, le carboxyméthylmaltoheptaose, le carboxyméthylmaltooactose et le carboxyméthylisomaltotriose.

Dans un mode de réalisation, l'insuline est une insuline humaine recombinante telle que décrite dans la Pharmacopée Européenne et la Pharmacopée américaine ?,

Dans un mode de réalisation, l'insuline est une insuline analogue choisie dans le groupe constitué par l'insuline lispro (Humalog^{®}), l'insuline aspart (Novolog^{®} , Novorapid^{®}) et l'insuline glulisine (Apidra^{®}).

Dans un mode de réalisation, les ratios molaires oligosaccharide/insuline sont compris entre 0,2 et 7.

Dans un mode de réalisation, les ratios molaires sont compris entre 0,3 et 5.

Dans un mode de réalisation, les ratios molaires sont compris entre 0,6 et 4.

Dans un mode de réalisation, les ratios molaires sont compris entre 1 et 3.

Dans un mode de réalisation, les ratios molaires ont compris entre 1,2 et 3.

Dans un mode de réalisation, le ratio molaire est égal à 1.

Dans un mode de réalisation, le ratio molaire est égal à 2.

Dans un mode de réalisation, les ratios massiques oligosaccharide/insuline sont compris entre 0,4 et 10.

Dans un mode de réalisation, les ratios massiques sont compris entre 0,6 et 7.

Dans un mode de réalisation, les ratios massiques sont compris entre 1,2 et 5.

Dans un mode de réalisation, les ratios massiques sont compris entre 1,6 et 4.

Dans un mode de réalisation, les ratios massiques sont compris entre 2 et 4.

Dans un mode de réalisation, la concentration en oligosaccharides fonctionnalisés est comprise entre 1,4 et 35 mg/mL.

Dans un mode de réalisation, la concentration en oligosaccharides fonctionnalisés est comprise entre 2,1 et 25 mg/mL.

Dans un mode de réalisation, la concentration en oligosaccharides fonctionnalisés est comprise entre 4,2 et 18 mg/mL.

Dans un mode de réalisation, la concentration en oligosaccharides fonctionnalisés est comprise entre 5,6 et 14 mg/mL.

Dans un mode de réalisation, la concentration en oligosaccharides fonctionnalisés est comprise entre 7 et 14 mg/mL.

Dans un mode de réalisation, la concentration en composé anionique est comprise entre 5 et 150 mM. Dans un mode de réalisation, la concentration en composé polyanionique est comprise entre 5 et 100 mM.

Dans un mode de réalisation, la concentration en composé polyanionique est comprise entre 5 et 75 mM.

Dans un mode de réalisation, la concentration en composé polyanionique est comprise entre 5 et 50 mM.

Dans un mode de réalisation, la concentration en composé polyanionique est comprise entre 5 et 30 mM.

Dans un mode de réalisation, la concentration en composé polyanionique est comprise entre 5 et 20 mM.

Dans un mode de réalisation, la concentration en composé polyanionique est comprise entre 5 et 10 mM.

Dans un mode de réalisation, la concentration en composé polyanionique e est comprise entre 1 et 30 mg/mL.

Dans un mode de réalisation, la concentration en composé polyanionique est comprise entre 1,5 et 25 mg/mL.

Dans un mode de réalisation, la concentration en composé polyanionique est comprise entre 2 et 25 mg/mL.

Dans un mode de réalisation, la concentration en composé polyanionique est comprise entre 2 et 10 mg/mL.

Dans un mode de réalisation, la concentration en composé polyanionique est comprise entre 2 et 8 mg/mL.

Dans un mode de réalisation, l'oligosaccharide est le Dextraneméthylcarboxylate de sodium modifié par le phénylalaninate de sodium, q = 4, i = 1,1, n = 0,45.

Dans un mode de réalisation, l'oligosaccharide est le Dextraneméthylcarboxylate de sodium modifié par le phénylalaninate de sodium, q = 4, i = 1,65, n = 0,39.

Dans un mode de réalisation, l'oligosaccharide est le Dextraneméthylcarboxylate de sodium modifié par le phénylalaninate de sodium, q = 4, i = 2,0, n = 0,5.

Dans un mode de réalisation comparative l'oligosaccharide est le Dextraneméthylcarboxylate de sodium modifié par le phénylalaninate de sodium, q = 4, i = 0,7, n = 0,57.

Dans un mode de réalisation, l'oligosaccharide est le Dextraneméthylcarboxylate de sodium modifié par le phénylalaninate de sodium, q = 4, i - 1,72, n = 0,42.

Dans un mode de réalisation comparative l'oligosaccharide est le Dextraneméthylcarboxylate de sodium modifié par le phénylalaninate de sodium, q = 4, i = 2,1, n = 0,6.

Dans un mode de réalisation, le composé polyanionique est le dextraneméthylcarboxylate de sodium

Dans un mode de réalisation, le composé polyanionique est le citrate de sodium.

La composition peut de plus être réalisée par simple mélange d'une solution aqueuse d'insuline humaine ou analogue et d'une solution aqueuse d'oligosaccharide.

Dans un mode de réalisation, la composition peut être réalisée par simple mélange d'une solution aqueuse d'insuline humaine ou analogue, d'une solution aqueuse d'oligosaccharide et de composé polyanionique en solution ou sous forme de lyophilisat.

Dans un mode de réalisation, la composition peut être réalisée par simple mélange d'une solution aqueuse d'insuline humaine ou analogue et d'oligosaccharide sous forme de lyophilisat.

Dans un mode de réalisation, la composition peut être réalisée par simple mélange d'une solution aqueuse d'insuline humaine ou analogue, d'oligosaccharide sous forme de lyophilisat et de composé polyanionique en solution ou sous forme de lyophilisat.

De préférence cette composition est sous forme d'une solution injectable.

Dans un mode de réalisation, la concentration en insuline humaine ou analogue est comprise entre 240 et 3000 µM (40 à 500 UI/mL).

Dans un mode de réalisation, la concentration en insuline humaine ou analogue est comprise entre 600 et 1200 µM (100 à 200 UI/mL).

Dans un mode de réalisation, la concentration en insuline humaine ou analogue est 500 µM (100 UI/mL).

Dans un mode de réalisation, la concentration en insuline humaine ou analogue est 1200 µM (200 UI/mL).

Dans un mode de réalisation, la concentration en insuline des solutions est de 600 µM soit 100 UI/mL.

Dans un mode de réalisation, la concentration en insuline humaine ou analogue de 600 µM peut être réduite par simple dilution, en particulier pour les applications pédiatriques.

L'invention concerne également une formulation pharmaceutique selon l'invention, caractérisée en ce qu'elle est obtenue par séchage et/ou lyophilisation.

Dans le cas des libérations locale et systémique, les modes d'administration envisagés sont par voie intraveineuse, sous-cutanée, intradermique ou intramusculaire.

Les voies d'administration transdermique, orale, nasale, vaginale, oculaire, buccale, pulmonaire sont également envisagées.

L'invention concerne également l'utilisation d'une composition selon l'invention pour la formulation d'une solution d'insuline humaine ou analogue de concentration de 100 UI/mL destinée aux pompes à insuline implantables ou transportables.

L'invention concerne également l'utilisation d'une composition selon l'invention pour la formulation d'une solution d'insuline humaine ou analogue de concentration de 200 UI/mL destinée aux pompes à insuline implantables ou transportables.

### Exemples

### AA. Oligosaccharides selon l'invention

| | SUBSTITUANTS | |
|---|---|---|
| OLIGOSACCHARIDES | -F-R | NOM USUEL |
| | -F-R-Phe | |
| **Oligosaccharide 1** | 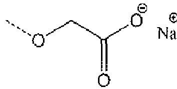 | Dextraneméthylcarboxylate de sodium modifié par le phénylalaninate de sodium |
| q : 4 | | |
| i : 1,1 | | |
| n : 0,45 | | |
| **Oligosaccharide 2** | | |
| q : 4 | | |
| i : 1,65 | | |
| n : 0,39 | | |
| **Oligosaccharide 3** | | |
| q : 4 | | |
| i : 2,0 | 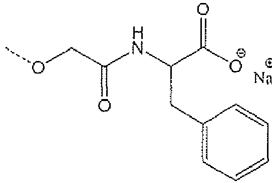 | |
| n : 0,5 | | |
| **Oligosaccharide** 4 | | |
| q : 4 | | |
| i : 0,7 | | |
| n : 0,57 | | |
| **Oligosaccharide 5** | | |
| q : 4 | | |
| i : 1,72 | | |
| n : 0,42 | | |
| **Oligosaccharide 6** | | |
| q : 4 | | |
| i : 2,1 | | |
| n : 0,6 | | |

### AA1. Oligosaccharide 1 : Dextraneméthylcarboxylate de sodium fonctionnalisé par le L-phénylalaninate de sodium.

40 g (0,74 mol de fonctions hydroxyles) de dextrane de masse molaire moyenne en poids 1 kg/mol (DP = 4, Pharmacosmos) et 115 g (0,99 mol) de chloroacétate de sodium sont dissouts dans l'eau à 65°C. A cette solution sont ajoutés goutte à goutte 123 mL de NaOH 10 N (1,23 mol) puis le mélange est chauffé à 65°C. Le mélange est ensuite dilué avec de l'eau, neutralisé avec de l'acide acétique puis purifié par ultrafiltration sur membrane PES de 1 kDa contre de l'eau. La concentration en oligosaccharide de la solution finale est déterminée par extrait sec, puis un dosage acide/base dans un mélange eau/acétone 50/50 (V/V) est effectué pour déterminer la fraction molaire moyenne de méthylcarboxylates de sodium.
D'aprés l'extrait sec: [oligosaccharide] = 37,6 mg/g

D'après le dosage acide/base, la fraction molaire moyenne de méthylcarboxylates de sodium est de 1,1.

La solution de dextraneméthylcarboxylate de sodium est acidifiée sur une résine Purolite (anionique) pour obtenir l'acide dextraneméthylcarboxylique qui est ensuite lyophilisé pendant 18 heures.

12 g d'acide dextraneméthylcarboxylique (61 mmol de fonctions acide méthylcarboxylique) sont solubilisés dans le DMF puis refroidis à 0°C. Un mélange de phénylalaninate d'éthyle, sel d'hydrochlorure (Bachem) (6 g, 26 mmol) dans du DMF est préparé. 2,6 g de triéthylamine (26 mmol) sont ajoutés à ce mélange. Une solution de NMM (6,1 g, 61 mmol) et de EtOCOCI (δ,6 g, 61 mmol) est ensuite ajoutée au mélange à 0°C. La solution de phénylalaninate d'éthyle est ensuite ajoutée et le mélange agité à 10°C. Une solution aqueuse d'imidazole est ajoutée puis le mélange chauffé à 30°C. Le milieu est dilué avec de l'eau puis la solution obtenue est purifiée par ultrafiltration sur membrane PES de 1 kDa contre NaOH 0,1 N, NaCl 0,9% et de l'eau. La concentration en oligosaccharide de la solution finale est déterminée par extrait sec. Un échantillon de solution est lyophilisé et analysé par RMN ¹H dans D₂O pour déterminer la fraction molaire moyenne de méthylcarboxylates de sodium fonctionnalisés par le L-phénylalaninate de sodium.
D'aprés l'extrait sec: [Oligosaccharide 1] = 20,8 mg/g

D'après la RMN ¹H : la fraction molaire moyenne de méthylcarboxylates de sodium fonctionnalisés par le L-phénylalaninate de sodium est de 0,45.

### AA2. Oligosaccharide 2 : Dextraneméthylcarboxylate de sodium fonctionnalisé par le L-phénylalaninate de sodium.

40 g (0,74 mol de fonctions hydroxyles) de dextrane de masse molaire moyenne en poids 1 kg/mol (DP = 4, Pharmacosmos) et 144 g (1,23 mol) de chloroacétate de sodium sont dissouts dans l'eau à 65°C. A cette solution sont ajoutés goutte à goutte 123 mL de NaOH 10 N (1,23 mol) puis le mélange est chauffé à 65°C pendant 90 minutes. 86,3 g (0,74 mol) de chloroacétate de sodium sont ensuite ajoutés au milieu réactionnel ainsi que 74,1 mL de NaOH 10 N (0,74 mol) au goutte à goutte et le chauffage poursuivi à 65°C. Le mélange est ensuite dilué avec de l'eau, neutralisé avec de l'acide acétique puis purifié par ultrafiltration sur membrane PES de 1 kDa contre de l'eau. La concentration en oligosaccharide de la solution finale est déterminée par extrait sec, puis un dosage acide/base dans un mélange eau/acétone 50/50 (V/V) est effectué pour déterminer la fraction molaire moyenne de méthylcarboxylates de sodium .
D'aprés l'extrait sec: [oligosaccharide] = 34,4 mg/g

D'après le dosage acide/base, la fraction molaire moyenne de méthylcarboxylates de sodium est de 1,65.

Par un procédé similaire à celui utilisé pour la préparation de l'oligosaccharide 1, un dextraneméthylcarboxylate de sodium fonctionnalisé par le L-phénylalaninate de sodium est obtenu.
D'aprés l'extrait sec: [Oligosaccharide 2] = 20,4 mg/g

D'après la RMN ¹H : la fraction molaire moyenne de méthylcarboxylates de sodium fonctionnalisés par le L-phénylalaninate de sodium est de 0,39.

### AA3. Oligosaccharide 3 : Dextraneméthylcarboxylate de sodium fonctionnalisé par le L-phénylalaninate de sodium.

L'oligosaccharide 3 est un dextraneméthylcarboxylate de sodium fonctionnalisé par le L-phénylalaninate de sodium obtenu à partir d'un dextrane de masse molaire moyenne en poids 1 kg/mol (DP = 4, Pharmacosmos)) selon le procédé décrit dans la demande de brevet FR 07/02316. La fraction molaire moyenne en méthylcarboxylates de sodium optionnellement fonctionnalisés par le L-phénylalaninate de de sodiumest 2,0. La fraction molaire moyenne de méthylcarboxylates de sodium fonctionnalisés par le L-phénylalaninate de sodium est 0,5.

Cet oligosaccharide est référencé polysaccharide 13 dans le document de priorité.

### AA4. Oligosaccharide 4 (comparatif) : Dextraneméthylcarboxylate de sodium fonctionnalisé par le L-phénylalaninate de sodium.

120 g (2,22 mol de fonctions hydroxyles) de dextrane de masse molaire moyenne en poids 1 kg/mol (DP = 4, Pharmacosmos) et 151 g (1,3 mol) de chloroacétate de sodium sont dissouts dans l'eau à 65°C. A cette solution sont ajoutés goutte à goutte 370 mL de NaOH 10 N (3,7 mol) puis le mélange est chauffé à 65°C. Le mélange est ensuite dilué avec de l'eau, neutralisé avec de l'acide acétique puis purifié par ultrafiltration sur membrane PES de 1 kDa contre de l'eau. La concentration en oligosaccharide de la solution finale est déterminée par extrait sec, puis un dosage acide/base dans un mélange eau/acétone 50/50 (V/V) est effectué pour déterminer la fraction molaire moyenne de méthylcarboxylates de sodium.
D'aprés l'extrait sec: [oligosaccharide] = 22,1 mg/g

D'après le dosage acide/base, la fraction molaire moyenne de méthylcarboxylates de sodium est de 0,7.

Par un procédé similaire à celui utilisé pour la préparation de l'oligosaccharide 1, un dextraneméthylcarboxylate de sodium fonctionnalisé par le L-phénylalaninate de sodium est obtenu.
D'aprés l'extrait sec: [Oligosaccharide 4] = 20,4 mg/g

D'après la RMN ¹H : la fraction molaire moyenne de méthylcarboxylates de sodium fonctionnalisés par le L-phénylalaninate de sodium est de 0,57.

### AA5. Oligosaccharide 5 : Dextraneméthylcarboxylate de sodium fonctionnalisé par le L-phénylalaninate de sodium.

L'oligosaccharide 5 est un dextraneméthylcarboxylate de sodium fonctionnalisé par le L-phénylalaninate de sodium obtenu à partir d'un dextrane de masse molaire moyenne en poids 1 kg/mol (DP = 4, Pharmacosmos) selon le procédé décrit dans la demande de brevet FR 07/02316. La fraction molaire moyenne en méthylcarboxylates de sodium optionnellement fonctionnalisés par le L-phénylalaninate de sodium est 1,72. La fraction molaire moyenne de méthylcarboxylates de sodium fonctionnalisés par le L-phénylalaninate de sodium est 0,42.

Cet oligosaccharide est référencé polysaccharide 12 dans le document de priorité.

### AA6. Oligosaccharide 6 (comparatif) : Dextraneméthylcarboxylate de sodium fonctionnalisé par le L-phénylalaninate de sodium.

L'oligosaccharide 6 est un dextraneméthylcarboxylate de sodium fonctionnalisé par le L-phénylalaninate de sodium obtenu à partir d'un dextrane de masse molaire moyenne en poids 1 kg/mol (DP = 4, Pharmacosmos)) selon le procédé décrit dans la demande de brevet FR 07/02316. La fraction molaire moyenne en méthylcarboxylates de sodium optionnellement fonctionnalisés par le L-phénylalaninate de de sodiumest 2,1. La fraction molaire moyenne de méthylcarboxylates de sodium fonctionnalisés par le L-phénylalaninate de sodium est 0,5.

### AB Polysaccharides contre exemples

### AB1. Polysaccharide 1 : Dextraneméthylcarboxylate de sodium fonctionnalisé par le L-phénylalaninate de sodium,

Le polysaccharide 1 est un dextraneméthylcarboxylate de sodium fonctionnalisé par le L-phénylalaninate de sodium obtenu à partir d'un dextrane de masse molaire moyenne en poids 10 kg/mol (DP = 39, Pharmacosmos) selon le procédé décrit dans la demande de brevet FR 07/02316. La fraction molaire moyenne en méthylcarboxylates de sodium est de 1,06. La fraction molaire moyenne de méthylcarboxylates de sodium fonctionnalisés par le **L-phénylalaninate** de sodium est de 0,43.

Ce polysaccharide correspond au polysaccharide 1 de la demande FR0901478.

### AB2. Polysaccharide 2 : Dextraneméthylcarboxylate de sodium fonctionnalisé par le L-phénylalaninate de sodium.

Le polysaccharide 2 est un dextraneméthylcarboxylate de sodium fonctionnalisé par le L-phénylalaninate de sodium obtenu à partir d'un dextrane de masse molaire moyenne en poids 5 kg/mol (DP = 19, Pharmacosmos) selon le procédé décrit dans la demande de brevet FR 07/02316. La fraction molaire moyenne en méthylcarboxylates de sodium est de 1,65. La fraction molaire moyenne de méthylcarboxylates de sodium fonctionnalisés par le L-phénylalaninate de sodium est de 0,39.

### AB3. Polysaccharide 3 : Dextraneméthylcarboxylate de sodium fonctionnalisé par le L-phénylalaninate de sodium.

Le polysaccharide 3 est un dextraneméthylcarboxylate de sodium fonctionnalisé par le L-phénylalaninate de sodium obtenu à partir d'un dextrane de masse molaire moyenne en poids 5 kg/mol (DP = 19, Pharmacosmos) selon le procédé décrit dans la demande de brevet FR 07/02316. La fraction molaire moyenne en méthylcarboxylates de sodium est de 1,10. La fraction molaire moyenne de méthylcarboxylates de sodium fonctionnalisés par le L-phénylalaninate de sodium est de 0,41.

### AB4. Polysaccharide 4 : Dextraneméthylcarboxylate de sodium fonctionnalisé par le L-phénylalaninate de sodium.

Le polysaccharide 4 est un dextraneméthylcarboxylate de sodium fonctionnalisé par le L-phénylalaninate de sodium obtenu à partir d'un dextrane de masse molaire moyenne en poids 10 kg/mol (DP = 39, Pharmacosmos) selon le procédé décrit dans la demande de brevet FR 07/02316. La fraction molaire moyenne en méthylcarboxylates de sodium est de 1,65. La fraction molaire moyenne de méthylcarboxylates de sodium fonctionnalisés par le L-phénylalaninate de sodium est de 0,39.

### AB5. Polysaccharide 5 : Dextraneméthylcarboxylate de sodium fonctionnalisé par le L-phénylalaninate de sodium.

Le polysaccharide 5 est un dextraneméthylcarboxylate de sodium fonctionnalisé par le L-phénylalaninate de sodium obtenu à partir d'un dextrane de masse molaire moyenne en poids 5 kg/mol (DP = 19, Pharmacosmos) selon le procédé décrit dans la demande de brevet FR 07/02316. La fraction molaire moyenne en méthylcarboxylates de sodium est de 1,10. La fraction molaire moyenne de méthylcarboxylates de sodium fonctionnalisés par le L-phénylalaninate de sodium est de 0,59,

### AC Composés polyanioniques

### AC1. Composé polyanionique 1 : Dextraneméthylcarboxylate de sodium.

40 g (0,74 mol de fonctions hydroxyles) de dextrane de masse molaire moyenne en poids 1 kg/mol (DP = 4, Pharmacosmos) et 144 g (1,23 mol) de chloroacétate de sodium sont dissouts dans l'eau à 60°C. A cette solution sont ajoutés goutte à goutte 123 mL de NaOH 10 N (1,23 mol) puis le mélange est chauffé à 60°C pendant 90 minutes, 86,3 g (0,74 mol) de chloroacétate de sodium sont ensuite ajoutés au milieu réactionnel ainsi que 74,1 mL de NaOH 10 N (0,74 mol) au goutte à goutte. Après 1h de chauffage, le mélange est dilué avec de l'eau, neutralisé avec del'acide acétique puis purifié par ultrafiltration sur membrane PES de 1 kDa contre de l'eau. La concentration en oligosaccharide de la solution finale est déterminée par extrait sec, puis un dosage acide/base dans un mélange eau/acétone 50/50 (V/V) est effectué pour déterminer la fraction molaire moyenne en méthylcarboxylates de sodium.
D'aprés l'extrait sec: [Composé polyanionique 1] = 34,4 mg/g

D'après le dosage acide/base, la fraction molaire moyenne en méthylcarboxylates de sodium est de 1,65.

### AC2. Composé polyanionique 2 : Maltotrioseméthylcarboxylate de sodium.

Le composé polyanionique 2 est un maltotrioseméthylcarboxylate de sodium obtenu par un procédé similaire à celui utilisé pour préparer le composé polyanionique 1. La fraction molaire moyenne en méthylcarboxylates de sodium est de 1,65.

### B Préparation des solutions

### B1. Solution d'insuline analogue rapide Novolog^{®} à 100 UI/ML.

Cette solution est une solution commerciale d'insuline aspart de Novo Nordisk vendue sous le nom de Novolog^{®}. Ce produit est une insuline aspart analogue rapide.

### B2. Solution d'insuline analogue rapide Humalog^{®} à 100 UI/mL.

Cette solution est une solution commerciale d'insuline lispro de Eli Lilly vendue sous le nom de Humalog^{®}. Ce produit est une insuline analogue rapide.

### B3. Solution d'insuline humaine régulière Actrapid^{®} à 100 UI/mL.

Cette solution est une solution commerciale d'insuline humaine de Novo Nordisk vendue sous le nom de Actrapid^{®}. Ce produit est une insuline humaine régulière.

### B4. Préparation des solutions d'excipients

Préparation d'une solution de citrate de sodium à 1,188 M.

Une solution de citrate de sodium est obtenue en solubilisant 9,0811 g de citrate de sodium (30,9 mmol) dans 25 mL d'eau dans une fiole jaugée. Le pH est ajusté exactement à 7,4 par ajout de 1 mL d'HCl 1 M. La solution est filtrée sur 0,22 µm.

Préparation d'une solution de *m*-crésol 130 mM.

Une solution de m-crésol est obtenue en solubilisant 14,114 g de m-crésol (130 mmol) dans 986,4 mL d'eau dans une fiole jaugée de 1 L.

Préparation d'une solution de m-crésol et glycérine (96,6 mM m-crésol et 566 mM glycérine).

73,3 g de la solution de m-crésol à 130 mM sont ajoutés à 5,26 g de glycérine et ensuite dilués par ajout de 22,25 g d'eau. La solution obtenue de m-crésol et glycérine est homogénéisée pendant 30 minutes puis filtrée sur une membrane 0,22 µm.

Préparation d'une solution de Tween 20 à 32,7 mM.

Une solution de Tween 20 est obtenue en solubilisant 2,0079 g de Tween 20 (1,636 mmol) dans 50 mL d'eau dans une fiole jaugée. La solution est filtrée sur une membrane 0,22 µm.

### 85. Préparation d'une solution d'insuline humaine à 500 UI/mL.

15 g d'eau sont ajoutés à 563,6 mg d'insuline humaine, puis le pH est abaissé à pH acide par ajout de 5,98 g d'HCl 0,1 N. Après solubilisation complète de l'insuline à pH acide, la solution est neutralisée à pH 7,2 par ajout de 8,3 mL de NaOH 0,1 N. La concentration est ensuite ajustée à 500 UI/mL par ajout de 0,76 g d'eau. La solution est enfin filtrée sur une membrane 0,22 µm.

### B6. Préparation d'une solution d'insuline humaine à 100 UI/mL en présence de l'oligosaccharide 2 et du composé polyanionique 1.

Pour un volume final de 100 mL de formulation avec un ratio massique [oligosaccharide 2]/[composé polyanionique 1J/[insuline] de 2/2/1, les différents réactifs sont mélangés en quantités précisées ci-dessous;

| | |
|---|---|
| Insuline humaine à 500 UI/mL | 20 mL |
| Solution de composé polyanionique 1 à 34,74 mg/mL | 21,01 mL |
| Solution 96,6 mM m-crésol/566 mM glycérine | 30 mL |
| Eau | 28,99 mL |
| Lyophilisat d'oligosaccharide 2 | 730 mg |
| Le pH final est de 7,4 ± 0,4. | |

Cette solution limpide est filtrée sur une membrane 0,22 µm puis est placée à +4°C.

### B7. Préparation d'une solution d'insuline humaine à 100 UI/mL en présence d'oligosaccharide 2 et de 9,3 mM de citrate.

Pour un volume final de 100 mL de formulation avec un ratio massique [oligosaccharide 2]/[insuline] de 2, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM *m*-crésol/566 mM glycérine | 30 mL |
| Eau | 28,95 mL |
| Solution de citrate de sodium à 1,188 M | 785 µL |
| Le pH final est de 7,4 ± 0,4. | |

Cette solution limpide est filtrée sur une membrane 0,22 µm puis est placée à +4°C.

### B8. (comparatif) Préparation d'une solution d'insuline lispro à 100 UI/mL en présence d'oligosaccharide 6.

Pour un volume final de 100 mL de formulation, avec un ratio massique [oligosaccharide 6]/[insuline lispro] de 2,0, les différents réactifs sont additionnés dans les quantités spécifiées ci- dessous:

| | |
|---|---|
| Oligosaccharide 6 sous forme lyophilisée | 730 mg |
| Solution commerciale d'Humalog à 100 UI/mf | 100 mL |

Le pH final est ajusté à 7,4 ± 0,4.

La solution limpide est filtrée sur une membrane 0,22 µm et stockée à 4°C.

### B9. Préparation d'une solution d'insuline lispro à 100 UI/mL en présence d'oligosaccharide 2 et de 9,3 mM de citrate.

Pour un volume final de 100 mL de formulation, avec un ratio massique [oligosaccharide 2]/[insuline lispro] de 2,0 et une concentration de 9,3 mM de citrate, les différents réactifs sont additionnés dans les quantités spécifiées ci-dessous :

| | |
|---|---|
| Oligosaccharide 2 sous forme lyophilisée | 730 mg |
| Solution commerciale d'Humalog à 100 UI/ml | 100 mL |
| Solution de citrate de sodium à 1,188 M | 785 µL |

Le pH final est ajusté à 7,4 ± 0,4. A cette solution peuvent éventuellement être rajoutés 25 µL de la solution de Tween 20 à 32,7mM (concentration finale en Tween 20 = 8 µM).

La solution limpide est filtrée sur une membrane 0,22 µm et stockée à 4°C.

### B10. Préparation d'une solution d'insuline analogue lispro à 100 UI/mL en présence d'oligosaccharide 2 et de 6 mM de citrate.

Pour un volume final de 100mL de formulation, avec un ratio massique [oligosaccharide 2]/[insuline lispro] de 2,0 et une concentration de 6 mM de citrate, les différents réactifs sont additionnés dans les quantités spécifiées ci-dessous :

| | |
|---|---|
| Oligosaccharide 2 sous forme lyophilisée | 730 mg |
| Solution commerciale Humalog^{®} 100 UI/ml | 100 mL |
| Solution de citrate de sodium à 1,188 M | 506 µL |

Le pH final est ajusté à 7,4 ± 0,4.

La solution limpide est filtrée sur une membrane 0,22 µm et stockée à 4°C.

### B11. Préparation d'une solution d'insuline analogue lispro à 100 UI/mL en présence d'oligosaccharide 1 et de 9,3 mM de citrate.

Pour un volume final de 100 mL de formulation, avec un ratio massique [oligosaccharide 1]/[lispro] de 2,0 et une concentration de 9,3 mM de citrate, les différents réactifs sont additionnés dans les quantités spécifiées ci-dessous :

| | |
|---|---|
| Oligosaccharide 1 sous forme lyophilisée | 730 mg |
| Solution commerciale Humalog^{®} 100 UI/ml | 100 mL |
| Solution de citrate de sodium à 1,188 M | 785 µL |

Le pH final est ajusté à 7,4 ± 0,4. La solution limpide est filtrée sur une membrane 0,22 µm et stockée à 4°C.

### B12, Préparation d'une solution d'insuline lispro à 100 UI/mL en présence d'oligosaccharide 1 et de 18,6 mM de citrate.

Pour un volume final de 100mL. de formulation, avec un ratio massique [oligosaccharide 1]/[lispro] de 2,0 et une concentration de 18,6 mM de citrate, les différents réactifs sont additionnés dans les quantités spécifiées ci-dessous :

| | |
|---|---|
| Oligosaccharide 1 sous forme lyophilisée | 730 mg |
| Solution commerciale Humalog^{®} 100 UI/ml | 100 mL |
| Solution de citrate de sodium à 1,188 M | 1570 µL |

Le pH final est ajusté à 7,4 ± 0,4. La solution limpide est filtrée sur une membrane 0,22 pm et stockée à 4°C.

### B13. Préparation d'une solution d'insuline lispro à 100 UI/mL en présence d'oligosaccharide 2 et de 7,3 mg/mL de composé polyanionique 1.

Pour un volume final de 100mL de formulation, avec un ratio massique [oligosaccharide 2]/[composé polyanionique 1]/[lispro] de 2/2/1, les différents réactifs sont additionnés dans les quantités spécifiées ci-dessous :

| | |
|---|---|
| Oligosaccharide 2 sous forme lyophilisée | 730 mg |
| Composé polyanionique 1 sous forme lyophilisée | 730 mg |
| Solution commerciale Humalog^{®} 100 UI/ml | 100 mL |

Le pH final est ajusté à 7,4 ± 0,4. La solution limpide est filtrée sur une membrane 0,22 µm et stockée à 4°C.

### B14. Préparation d'une solution d'insuline lispro à 100 UI/mL en présence d'oligosaccharide 2 et de 14,6 mg/mL de composé polyanionique 1.

Pour un volume final de 100 mL de formulation, avec un ratio massique [oligosaccharide 2]/[composé polyanionique 1]/[ lispro]de 2/4/1, les différents réactifs sont additionnés dans les quantités spécifiées ci-dessous :

| | |
|---|---|
| Oligosaccharide 2 sous forme lyophilisée | 730 mg |
| Composé polyanionique 1 sous forme lyophilisée | 1460 mg |
| Solution commerciale Humalog^{®} 100 UI/ml | 100 mL |

Le pH final est ajusté à 7,4 ± 0,4. La solution limpide est filtrée sur une membrane 0,22 µm et stockée à 4°C.

### B15. Préparation d'une solution d'insuline lispro à 100 UI/mL en présence d'oligosaccharide 2 à 14,6 mg/mL.

Pour un volume final de 100 mL de formulation, avec un ratio massique [oligosaccharide 2]/[insuline lispro] de 4, les différents réactifs sont additionnés dans les quantités spécifiées :

| | |
|---|---|
| Oligosaccharide 2 sous forme lyophilisée | 1460 mg |
| Solution commerciale Humalog^{®} 100 UI/ml | 100 mL |

Le pH final est ajusté à 7,4 ± 0,4. La solution limpide est filtrée sur une membrane 0,22 µm et stockée à 4°C.

### B16. Préparation d'une solution d'insuline analogue lispro à 100 UI/mL en présence d'oligosaccharide 2 et de 80 mM de tartrate de sodium.

Pour un volume final de 100 mL de formulation, avec un ratio massique [oligosaccharide 2]/[insuline lispro] de 2,0 et une concentration de 80 mM de tartrate de sodium, les différents réactifs sont additionnés dans les quantités spécifiées ci-dessous :

| | |
|---|---|
| Oligosaccharide 2 sous forme lyophilisée | 730 mg |
| Solution commerciale Humalog^{®} 100 UI/ml | 100 mL |
| Tartrate de sodium | 1,552 g |

Pour le tartrate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B17. Préparation d'une solution d'insuline analogue lispro à 100 UI/mL en présence d'oligosaccharide 2 et de 60 mM de phosphate.

Pour un volume final de 100 mL de formulation, avec un ratio massique [oligosaccharide 2]/[insuline lispro] de 2,0 et une concentration de 60 mM de phosphate, les différents réactifs sont additionnés dans les quantités spécifiées ci-dessous :

| | |
|---|---|
| Oligosaccharide 2 sous forme lyophilisée | 730 mg |
| Solution commerciale Humalog^{®} 100 UI/mf | 100 mL |
| Na₃HPO₄,12H₂O | 2,148 g |

Pour le phosphate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B18. Préparation d'une solution d'insuline analogue lispro à 100 UI/mL en présence d'oligosaccharide 2 et de 80 mM d'aspartate de sodium,

Pour un volume final de 100mL de formulation, avec un ratio massique [oligosaccharide 2]/[insuline lispro] de 2,0 et une concentration de 80 mM d'aspartate de sodium, les différents réactifs sont additionnés dans les quantités spécifiées ci-dessous :

| | |
|---|---|
| Oligosaccharide 2 sous forme lyophilisée | 730 mg |
| Solution commerciale Humalog^{®} 100 UI/ml | 100 mL |
| Aspartate de sodium | 1.416 g |

Pour l'aspartate, on peut utiliser, la forme acide ou la forme basique sous forme de sel de sodium, de sel de potassium ou d'un autre sel compatible avec une formulation injectable,

Le pH final est ajusté à 7,4 ± 0,4.

La solution limpide est filtrée sur une membrane 0,22 µm et stockée à 4°C.

### B19. Préparation d'une solution d'insuline analogue lispro à 100 UI/mL en présence d'oligosaccharide 2 et de 100 mM de glutamate de sodium.

Pour un volume final de 100 mL de formulation, avec un ratio massique [oligosaccharide 2]/[insuline lispro] de 2,0 et une concentration de 100 mM de glutamate de sodium, les différents réactifs sont additionnés dans les quantités spécifiées ci-dessous :

| | |
|---|---|
| Oligosaccharide 2 sous forme lyophilisée | 730 mg |
| Solution commerciale Humalog^{®} 100 UI/ml | 100 mL |
| Glutamate de sodium | 1,691 g |

Pour le glutamate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B20. Préparation d'une solution d'insuline analogue lispro à 100 UI/mL en présence d'oligosaccharide 2 et de 60 mM d'acide malique.

Pour un volume final de 100 mL de formulation, avec un ratio massique [oligosaccharide 2]/[insuline lispro] de 2,0 et une concentration de 60 mM d'acide malique, les différents réactifs sont additionnés dans les quantités spécifiées ci-dessous :

| | |
|---|---|
| Oligosaccharide 2 sous forme lyophilisée | 730 mg |
| Solution commerciale Humalog^{®} 100 UI/ml | 100 mL |
| Acide malique | 0,805 g |

Pour l'acide malique, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B21. Préparation d'une solution d'insuline analogue lispro à 100 UI/mL en présence d'oligosaccharide 2 et de 80 mM de succinate de sodium.

Pour un volume final de 100 mL de formulation, avec un ratio massique [oligosaccharide 2]/[insuline lispro] de 2,0 et une concentration de 80 mM de succinate de sodium, les différents réactifs sont additionnés dans les quantités spécifiées ci-dessous :

| | |
|---|---|
| Oligosaccharide 2 sous forme lyophilisée | 730 mg |
| Solution commerciale Humalog^{®} 100 UI/ml | 100 mL |
| Succinate de sodium | 1,296 g |

Pour le succinate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B22. Préparation d'une solution d'insuline analogue lispro à 100 UI/mL en présence d'oligosaccharide 2 et de 50 mM d'adipate de sodium.

Pour un volume final de 100mL de formulation, avec un ratio massique [oligosaccharide 2]/[insuline lispro] de 2,0 et une concentration de 50mM d'adipate de sodium, les différents réactifs sont additionnés dans les quantités spécifiées ci-dessous :

| | |
|---|---|
| Oligosaccharide 2 sous forme lyophilisée | 730 mg |
| Solution commerciale Humalog^{®} 100 UI/ml | 100 mL |
| Adipate de sodium | 0,951g |

Pour l'adipate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 pm et stockée à 4°C.

### B23. Préparation d'une solution d'insuline analogue lispro à 100 UI/mL en présence d'oligosaccharide 2 et de 80mM d'ascorbate de sodium.

Pour un volume final de 100mL de formulation, avec un ratio massique [oligosaccharide 2]/[insuline lispro] de 2,0 et une concentration de 80 mM d'ascorbate de sodium, les différents réactifs sont additionnés dans les quantités spécifiées ci-dessous :

| | |
|---|---|
| Oligosaccharide 2 sous forme lyophilisée | 730 mg |
| Solution commerciale Humalog^{®} 100 UI/ml | 100 mL |
| Ascorbate de sodium | 1,585 g |

Pour l'ascorbate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B24. Préparation d'une solution d'insuline analogue lispro à 100 UI/mL en présence d'oligosaccharide 2 et de 10 mM d'oxalate de sodium.

Pour un volume final de 100 mL de formulation, avec un ratio massique [oligosaccharide 2]/[insuline lispro] de 2,0 et une concentration de 10 mM d'oxalate de sodium, les différents réactifs sont additionnés dans les quantités spécifiées ci-dessous :

| | |
|---|---|
| Oligosaccharide 2 sous forme lyophilisée | 730 mg |
| Solution commerciale Humalog^{®} 100 UI/ml | 100 mL |
| Oxalate de sodium | 134 mg |

Pour l'oxalate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B25. Préparation d'une solution d'insuline lispro à 100 UI/mL en présence d'oligosaccharide 2 et de 14,6 mg/mL d'acide polyglutamique.

Pour un volume final de 100 mL de formulation, avec un ratio massique [oligosaccharide 2]/[acide polyglutamique]/[insuline lispro] de 2/4/1, les différents réactifs sont additionnés dans les quantités spécifiées ci-dessous :

| | |
|---|---|
| Oligosaccharide 2 sous forme lyophilisée | 730 mg |
| Acide polyglutamique sous forme lyophilisée | 1460 mg |
| Solution commerciale Humalog^{®} 100 UI/ml | 100 mL |

Pour l'acide polyglutamique, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B26. Préparation d'une solution d'insuline lispro à 100 UI/mL en présence d'oligosaccharide 2 et de 14,6 mg/mL d'acide polyaspartique.

Pour un volume final de 100 mL de formulation, avec un ratio massique [oligosaccharide 2]/[acide polyaspartique]/[insuline lispro] de 2/4/1, les différents réactifs sont additionnés dans les quantités spécifiées ci-dessous :

| | |
|---|---|
| Oligosaccharide 2 sous forme lyophilisée | 730 mg |
| Acide polyaspartique sous forme lyophilisée | 1460 mg |
| Solution commerciale Humalog^{®} 100 UI/ml | 100 mL |

Pour l'acide polyaspartique, on peut utiliser, la forme acide ou la forme basique sous forme de sel de sodium, de sel de potassium ou d'un autre sel compatible avec une formulation injectable.

Le pH final est ajusté à 7,4 1 0,4. La solution limpide est filtrée sur une membrane 0,22 µm et stockée à 4°C.

### B27. Préparation d'une solution d'insuline lispro à 100 UI/mL en présence d'oligosaccharide 2 et de 14,6 mg/mL de composé polyanionique 2.

Pour un volume final de 100 mL de formulation, avec un ratio massique [oligosaccharide 2]/[composé polyanionique 2]/[insuline lispro] de 2/4/1, les différents réactifs sont additionnés dans les quantités spécifiées ci-dessous :

| | |
|---|---|
| Oligosaccharide 2 sous forme lyophilisée | 730 mg |
| Composé polyanionique 2 sous forme lyophilisée | 1460 mg |
| Solution commerciale Humalog^{®} 100 UI/ml | 100 mL |

Le composé polyanionique 2 peut être utilisé sous la forme acide ou la forme basique sous forme de sel de sodium, de sel de potassium ou d'un autre sel compatible avec une formulation injectable,

Le pH final est ajusté à 7,4 ± 0,4. La solution limpide est filtrée sur une membrane 0,22 µm et stockée à 4°C.

### B28. Préparation d'une solution d'insuline lispro à 100 UI/mL en présence d'oligosaccharide 2 et de 7,3 mg/mL de triphosphate de sodium.

Pour un volume final de 100 mL de formulation, avec un ratio massique [oligosaccharide 2]/[triphosphate]/[insuline lispro] de 2/2/1, les différents réactifs sont additionnés dans les quantités spécifiées ci-dessous :

| | |
|---|---|
| Oligosaccharide 2 sous forme lyophilisée | 730 mg |
| Triphosphate ou polyphosphatede sodium | 730 mg |
| Solution commerciale Humalog^{®} 100 UI/ml | 100 mL |

Pour le triphosphate ou polyphosphate, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### 829. Préparation d'une solution d'insuline lispro à 100 UI/mL en présence d'oligosaccharide 2 et de 14,6 mg/mL de poly(acide acrylique).

Pour un volume final de 100 mL de formulation, avec un ratio massique [oligosaccharide 2]/[poly(acide acrylique)]/[insuline lispro] de 2/4/1, les différents réactifs sont additionnés dans les quantités spécifiées ci-dessous :

| | |
|---|---|
| Oligosaccharide 2 sous forme lyophilisée | 730 mg |
| Poly(acide acrylique) sous forme lyophilisée | 1460 mg |
| Solution commerciale Humalog^{®} 100 UI/ml | 100 mL |

Pour le poly(acide acrylique), on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B30. Préparation d'une solution d'insuline lispro à 100 UI/mL en présence d'oligosaccharide 2 et de 14,6 mg/mLd'alginate de sodium (bas poids moléculaire).

Pour un volume final de 100mL de formulation, avec un ratio massique [oligosaccharide 2]/[alginate de sodium]/[insuline lispro] de 2/4/1, les différents réactifs sont additionnés dans les quantités spécifiées ci-dessous :

| | |
|---|---|
| Oligosaccharide 2 sous forme lyophilisée | 730 mg |
| Alginate de sodium | 1460 mg |
| Solution commerciale Humalog^{®} 100 UI/ml | 100 mL |

Pour l'alginate, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B31. Préparation d'une solution d'insuline lispro à 100 UI/mL en présence d'oligosaccharide 2 et de 21,9 mg/mL de polymère à base d'acide glucuronique.

Pour un volume final de 100mL de formulation, avec un ratio massique [oligosaccharide 2]/[polymère à base d'acide glucuronique]/[insuline lispro] de 2/6/1, les différents réactifs sont additionnés dans les quantités spécifiées ci-dessous :

| | |
|---|---|
| Oligosaccharide 2 sous forme lyophilisée | 730 mg |
| Polymère à base d'acide glucuronique | 2190 mg |
| Solution commerciale Humalog^{®} 100 UI/ml | 100 mL |

Pour les polymères à base d'acide glucuronique, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B32. Préparation d'une solution d'insuline lispro à 100 UI/mL en présence d'oligosaccharide 2 et de 21,9 mg/mL de polymère à base d'acide galacturonique,

Pour un volume final de 100mL de formulation, avec un ratio massique [oligosaccharide 2]/[polymère à base d'acide galacturonique]/[insuline lispro] de 2/6/1, les différents réactifs sont additionnés dans les quantités spécifiées ci-dessous :

| | |
|---|---|
| Oligosaccharide 2 sous forme lyophilisée | 730 mg |
| polymère à base d'acide galacturonique | 2190 mg |
| Solution commerciale Humalog^{®} 100 UI/ml | 100 mL |

Pour les polymères à base d'acide galacturonique, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### 833. Préparation d'une solution d'insuline lispro à 100 UI/mL en présence d'oligosaccharide 2 et de 21,9 mg/mL polymère à base d'acide hyaluronique.

Pour un volume final de 100 mL de formulation, avec un ratio massique [oligosaccharide 2]/[polymère à base d'acide hyaluronique]/[insuline lispro] de 2/6/1, les différents réactifs sont additionnés dans les quantités spécifiées ci-dessous :

| | |
|---|---|
| Oligosaccharide 2 sous forme lyophilisée | 730 mg |
| polymère à base d'acide hyaluronique | 2190 mg |
| Solution commerciale Humalog^{®} 100 UI/ml | 100 mL |

Pour les polymères à base d'acide hyaluronique, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm) et stockée à 4°C.

### B34. Préparation d'une solution d'insuline analogue lispro à 100 UI/mL en présence d'oligosaccharide 1 et de 80 mM de tartrate.

Pour un volume final de 100 mL de formulation, avec un ratio massique [oligosaccharide 1]/[insuline lispro] de 2,0 et une concentration de 80 mM de tartrate, les différents réactifs sont additionnés dans les quantités spécifiées ci-dessous :

| | |
|---|---|
| Oligosaccharide 1 sous forme lyophilisée | 730 mg |
| Solution commerciale Humalog^{®} 100 UI/ml | 100 mL |
| Tartrate de sodium | 1,552 g |

Pour le tartarte, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B35. Préparation d'une solution d'insuline analogue lispro à 100 UI/mL en présence d'oligosaccharide 1et de 60 mM de phosphate,

Pour un volume final de 100 mL de formulation, avec un ratio massique [oligosaccharide 1]/[insuline lispro] de 2,0 et une concentration de 60 mM de phosphate, les différents réactifs sont additionnés dans les quantités spécifiées ci-dessous :

| | |
|---|---|
| Oligosaccharide 1 sous forme lyophilisée | 730 mg |
| Solution commerciale Humalog^{®} 100 UI/ml | 100 mL |
| Na₂HPO₄,12H₂O | 2,148 g |

Pour le phosphate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### 836. Préparation d'une solution d'insuline analogue lispro à 100 UI/mL en présence d'oligosaccharide 1 et de 80 mM d'aspartate de sodium.

Pour un volume final de 100 mL de formulation, avec un ratio massique [oligosaccharide 1]/[insuline lispro] de 2,0 et une concentration de 80 mM d'aspartate de sodium, les différents réactifs sont additionnés dans les quantités spécifiées ci-dessous :

| | |
|---|---|
| Oligosaccharide1 sous forme lyophilisée | 730 mg |
| Solution commerciale Humalog^{®} 100 UI/ml | 100 mL |
| Aspartate de sodium | 1,416 g |

Pour l'aspartate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B37. Préparation d'une solution d'insuline analogue lispro à 100 UI/mL en présence d'oligosaccharide 1et de 100 mM de glutamate de sodium.

Pour un volume final de 100 mL de formulation, avec un ratio massique [oligosaccharide 1]/[insuline lispro] de 2,0 et une concentration de 100 mM de glutamate de sodium, les différents réactifs sont additionnés dans les quantités spécifiées ci-dessous :

| | |
|---|---|
| Oligosaccharide 1 sous forme lyophilisée | 730 mg |
| Solution commerciale Humalog^{®} 100 UI/ml | 100 mL |
| Glutamate de sodium | 1,691 g |

Pour le glutamate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B38. Préparation d'une solution d'insuline analogue lispro à 100 UI/mL en présence d'oligosaccharide 1 et de 60 mM d'acide malique.

Pour un volume final de 100 mL de formulation, avec un ratio massique [oligosaccharide 1]/[insuline lispro] de 2,0 et une concentration de 60 mM d'acide malique, les différents réactifs sont additionnés dans les quantités spécifiées ci-dessous :

| | |
|---|---|
| Oligosaccharide 1 sous forme lyophilisée | 730 mg |
| Solution commerciale Humalog^{®} 100 UI/ml | 100 mL |
| Acide malique | 0,805 g |

Pour l'acide malique, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### β39. Préparation d'une solution d'insuline analogue lispro à 100 UI/mL en présence d'oligosaccharide 1 et de 80 mM de succinate de sodium,

Pour un volume final de 100 mL de formulation, avec un ratio massique [oligosaccharide 1J/[insuline lispro] de 2,0 et une concentration de 80 mM de succinate de sodium, les différents réactifs sont additionnés dans les quantités spécifiées ci-dessous :

| | |
|---|---|
| Oligosaccharide 1 sous forme lyophilisée | 730 mg |
| Solution commerciale Humalog^{®} 100 VI/ml | 100 mL |
| Succinate de sodium | 1,296 g |

Pour le succinate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### 840. Préparation d'une solution d'insuline analogue lispro à 100 UI/mL en présence d'oligosaccharide 1 et de 50 mM d'adipate de sodium,

Pour un volume final de 100 mL de formulation, avec un ratio massique [oligosaccharide 1]/[insuline lispro] de 2,0 et une concentration de 50 mM d'adipate de sodium, les différents réactifs sont additionnés dans les quantités spécifiées ci-dessous :

| | |
|---|---|
| Oligosaccharide 1 sous forme lyophilisée | 730 mg |
| Solution commerciale Humalog^{®} 100 UI/ml | 100 mL |
| Adipate de sodium | 0,951 g |

Pour l'adipate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B41. Préparation d'une solution d'insuline analogue lispro à 100 UI/mL en présence d'oligosaccharide 1 et de 80 mM d'ascorbate de sodium.

Pour un volume final de 100 mL de formulation, avec un ratio massique [oligosaccharide 1]/[insuline lispro] de 2,0 et une concentration de 80 mM d'ascorbate de sodium, les différents réactifs sont additionnés dans les quantités spécifiées ci-dessous :

| | |
|---|---|
| Oligosaccharide 1 sous forme lyophilisée | 730 mg |
| Solution commerciale Humalog^{®} 100 UI/ml | 100 mL |
| Ascorbate de sodium | 1,585 g |

Pour l'ascorbate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B42. Préparation d'une solution d'insuline analogue lispro à 100 UI/mL en présence d'oligosaccharide 1 et de 10 mM d'oxalate de sodium.

Pour un volume final de 100 mL de formulation, avec un ratio massique [oligosaccharide 1]/[insuline lispro] de 2,0 et une concentration de 10 mM d'oxalate de sodium, les différents réactifs sont additionnés dans les quantités spécifiées ci-dessous :

| | |
|---|---|
| Oligosaccharide 1 sous forme lyophilisée | 730 mg |
| Solution commerciale Humalog^{®} 100 UI/ml | 100 mL |
| Oxalate de sodium | 134 mg |

Pour l'oxalate, on peut utiliser, la forme acide ou la forme basique sous forme de sel de sodium, de sel de potassium ou d'un autre sel compatible avec une formulation injectable.

Le pH final est ajusté à 7,4 ± 0,4.

La solution limpide est filtrée sur une membrane 0,22 µm et stockée à 4°C.

### B43. Préparation d'une solution d'insuline lispro à 100 UI/mL en présence d'oligosaccharide 1 et de 14,6 mg/mLd'acide polyglutamique.

Pour un volume final de 100 mL de formulation, avec un ratio massique [oligosaccharide 1]/[acide polyglutamique]/[insuline lispro] de 2/4/1, les différents réactifs sont additionnés dans les quantités spécifiées ci-dessous :

| | |
|---|---|
| Oligosaccharide 1 sous forme lyophilisée | 730 mg |
| Acide polyglutamique sous forme lyophilisée | 1460 mg |
| Solution commerciale Humalog^{®} 100 UIjml | 100 mL |

Pour l'acide polyglutamique, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B44. Préparation d'une solution d'insuline lispro à 100 UI/mL en présence d'oligosaccharide 1et de 14,6 mg/mL d'acide polyaspartique.

Pour un volume final de 100 mL de formulation, avec un ratio massique [oligosaccharide 1]/[acide polyaspartique]/[insuline lispro] de 2/4/1, les différents réactifs sont additionnés dans les quantités spécifiées ci-dessous :

| | |
|---|---|
| Oligosaccharide 1 sous forme lyophilisée | 730 mg |
| Acide polyaspartique sous forme lyophilisée | 1460 mg |
| Solution commerciale Humalog^{®} 100 UI/ml | 100 mL |

Pour l'acide polyaspartique, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B45. Préparation d'une solution d'insuline lispro à 100 UI/mL en présence d'oligosaccharide 1 et de 14,6 mg/mL de composé polyanionique 2.

Pour un volume final de 100 mL de formulation, avec un ratio massique [oligosaccharide 1]/[composé polyanionique 2]/[insuline lispro] de 2/4/1, les différents réactifs sont additionnés dans les quantités spécifiées ci-dessous :

| | |
|---|---|
| Oligosaccharide 1 sous forme lyophilisée | 730 mg |
| Composé polyanionique 2 sous forme lyophilisée | 1460 mg |
| Solution commerciale Humalog^{®} 100 UI/ml | 100 mL |

Le composé polyanionique 2 peut être utilisé sous la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B45. Préparation d'une solution d'insuline lispro à 100 UI/mL en présence d'oligosaccharide 1 et de 7,3 mg/mL de triphosphate de sodium.

Pour un volume final de 100 mL de formulation, avec un ratio massique [oligosaccharide 1]/[triphosphate]/[insuline lispro] de 2/2/1, les différents réactifs sont additionnés dans les quantités spécifiées ci-dessous :

| | |
|---|---|
| Oligosaccharide 1 sous forme lyophilisée | 730 mg |
| Triphosphate ou polyphosphate de sodium | 730 mg |
| Solution commerciale Humalog^{®} 100 UI/m! | 100 mL |

Pour le triphosphate, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B47. Préparation d'une solution d'insuline lispro à 100 UI/mL en présence d'oligosaccharide 1 et de 14,6 mg/mL de poly(acide acrylique).

Pour un volume final de 100 mL de formulation, avec un ratio massique [oligosaccharide 1]/[poly(acide acrylique)]/[insuline lispro] de 2/4/1, les différents réactifs sont additionnés dans les quantités spécifiées ci-dessous :

| | |
|---|---|
| Oligosaccharide 1 sous forme lyophilisée | 730 mg |
| Poly(acide acrylique) sous forme lyophilisée | 1460 mg |
| Solution commerciale Humalog^{®} 100 UI/ml | 100 mL |

Pour le poly(acide acrylique), on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### 848. Préparation d'une solution d'insuline lispro à 100 UI/mL en présence d'oligosaccharide 1 et de 14,6 mg/mL d'alginate de sodium (bas poids moléculaire).

Pour un volume final de 100 mL de formulation, avec un ratio massique [oligosaccharide 1]/[alginate de sodium]/[insuline lispro] de 2/4/1, les différents réactifs sont additionnés dans les quantités spécifiées ci-dessous :

| | |
|---|---|
| Oligosaccharide 1 sous forme lyophilisée | 730 mg |
| Alginate de sodium | 1460 mg |
| Solution commerciale Humalog^{®} 100 UI/ml | 100 mL |

Pour l'alginate, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B49. Préparation d'une solution d'insuline lispro à 100 UI/mL en présence d'oligosaccharide 1 et de 21,9 mg/mL de polymère à base d'acide glucuronique,

Pour un volume final de 100mL de formulation, avec un ratio massique [oligosaccharide 1]/[polymère à base d'acide glucuronique]/[insuline lispro] de 2/6/1, les différents réactifs sont additionnés dans les quantités spécifiées ci-dessous :

| | |
|---|---|
| Oligosaccharide 1 sous forme lyophilisée | 730 mg |
| Polymère à base d'acide glucuronique | 2190 mg |
| Solution commerciale Humalog^{®} 100 UI/ml | 100 mL |

Pour les polymères à base d'acide glucuronique, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B50. Préparation d'une solution d'insuline lispro à 100 UI/mL en présence d'oligosaccharide 1 et de 21,9 mg/mL de polymère à base d'acide galacturonique,

Pour un volume final de 100 mL de formulation, avec un ratio massique [oligosaccharide 1]/[polymère à base d'acide galacturonique]/[insuline lispro] de 2/6/1, les différents réactifs sont additionnés dans les quantités spécifiées ci-dessous :

| | |
|---|---|
| Oligosaccharide 1 sous forme lyophilisée | 730 mg |
| polymère à base d'acide galacturonique | 2190 mg |
| Solution commerciale Humalog^{®} 100 UI/ml | 100 mL |

Pour les polymères à base d'acide galacturonique, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B51. Préparation d'une solution d'insuline lispro à 100 UI/mL en présence d'oligosaccharide 1 et de 21,9 mg/mL de polymère à base d'acide hyaluronique.

Pour un volume final de 100 mL de formulation, avec un ratio massique [oligosaccharide 1]/[polymère à base d'acide hyaluroniquej/[insuline lispro] de 2/6/1, les différents réactifs sont additionnés dans les quantités spécifiées ci-dessous :

| | |
|---|---|
| Oligosaccharide 1 sous forme lyophilisée | 730 mg |
| polymère à base d'acide hyaluronique | 2190 mg |
| Solution commerciale Humalog^{®} 100 UI/ml | 100 mL |

Pour les polymères à base d'acide hyaluronique, on peut utiliser, la forme acide ou la forme basique sous forme de sel de sodium, de sel de potassium ou d'un autre sel compatible avec une formulation injectable.

Le pH final est ajusté à 7,4 ± 0,4. La solution limpide est filtrée sur une membrane 0,22 µm et stockée à 4°C.

### B52. Préparation d'une solution d'insuline analogue (insuline lispro) à 200 UI/mL.

La formulation commerciale d'insuline lispro (Humalog^{®}) a été concentrée en utilisant des tubes de centrifugation AMICON Ultra-15 avec une coupure à 3 kDa. Les tubes amicon on tout d'abord été rincés avec 12 mL d'eau déionisée. 12 mL de la formulation commerciale ont été centrifugés pendant 35 minutes à 4000 g à 20°C. Le volume du rétentat a été mesuré et la concentration estimée par le volume de rétentat. Tous les rétentats ont été mis en commun et la concentration globale a été estimée (> 200 UI/mL).

La concentration de cette solution de lispro concentrée a été ajustée à 200 UI/mL par addition de la formulation commerciale d'insuline lispro (Humalog^{®}). La formulation concentrée d'insuline lispro concentrée présente les mêmes concentrations en excipients (*m*-crésol, glycérine, phosphate) que la formulation commerciale à 100 UI/mL.

Le pH final est ajusté à 7,4 ± 0,4. La solution limpide est filtrée sur une membrane 0,22 µm et stockée à 4°C.

### B53. Préparation d'une solution d'insuline lispro à 200 UI/mL en présence d'oligosaccharide 2 à 14,6 mg/mL et de 9,3 mM de citrate.

Pour un volume final de 100 mL de formulation avec un ratio massique [oligosaccharide 2/Lispro] de 2, les différents réactifs sont mélangés en quantités précisées ci-dessous

| | |
|---|---|
| Insuline lispro à 200 UI/mL | 100 mL |
| Lyophilisat d'oligosaccharide 2 | 1460 mg |
| Solution de citrate de sodium à 1,188 M | 1570 µL |

Le pH final est ajusté à 7,4 ± 0,4.

La solution limpide est filtrée sur une membrane 0,22 µm et stockée à 4°C.

### 854. Préparation d'une solution d'insuline lispro à 200 UI/mL en présence d'oligosaccharide 2 à 14,6 mg/mL et de composé polyanionique 1 à 14,6 mg/mL.

Pour un volume final de 100 mL de formulation avec un ratio massique [(oligosaccharide 2/composé polyanionique 1/lispro] de 2/2/1, les différents réactifs sont mélangés en quantités précisées ci-dessous.

| | |
|---|---|
| Insuline lispro à 200 UI/mL | 100 mL |
| Lyophilisat d'oligosaccharide 2 | 1460 mg |
| Lyophilisat de composé polyanionique 1 | 1460 mg |

Le pH final est ajusté à 7,4 ± 0,4.

La solution limpide est filtrée sur une membrane 0,22 µm et stockée à 4°C.

### 855. Préparation d'une solution d'insuline lispro à 200 UI/mL en présence d'oligosaccharide 2 à 14,6 mg/mL et de composé polyanionique 1 à 29,2 mg/mL.

Pour un volume final de 100 mL de formulation avec un ratio massique [oligosaccharide 2/composé polyanionique 1/lispro] de 2/4/1, les différents réactifs sont mélangés en quantités précisées ci-dessous.

| | |
|---|---|
| Insuline lispro à 200 UI/mL | 100 mL |
| Lyophilisat d'oligosaccharide 2 | 1460 mg |
| Lyophilisat de composé polyanionique 1 | 2920 mg |

Le pH final est ajusté à 7,4 ± 0,4.

La solution limpide est filtrée sur une membrane 0,22 µm et stockée à 4°C.

### B56. : Préparation d'une solution d'insuline humaine à 100 UI/mL en présence d'oligosaccharide 2 et de 80 mM de tartrate.

Pour un volume final de 100 mL de formulation avec un ratio massique [oligosaccharide 2]/[insuline] de 2 et 80 mM de tartrate, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 m L |
| Solution 96,6 mM m-crésol/566 mM glycérine | 30 mL |
| Eau | 28,95 mL |
| Tartrate de sodium | 1,552 g |

Pour le tartrate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B57. Préparation d'une solution d'insuline humaine à 100 UI/mL en présence d'oligosaccharide 2 et de 80 mM de phosphate.

Pour un volume final de 100 mL de formulation avec un ratio massique[oligosaccharide 2]/[insuline] de 2 et 80 mM de phosphate, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Na₂HPO₄,12H₂O | 2,864 g |

Pour le phosphate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B58 Préparation d'une solution d'insuline humaine à 100 UI/mL en présence d'oligosaccharide 2 et de 80 mM d'aspartate,

Pour un volume final de 100 mL de formulation avec un ratio massique [oligosaccharide 2]/[insuline] de 2 et 80 mM d'aspartate, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Aspartate de sodium | 1,416 g |

Pour l'aspartate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B59. Préparation d'une solution d'insuline humaine à 100 UI/mL en présence d'oligosaccharide 2 et de 100 mM de glutamate.

Pour un volume final de 100 mL de formulation avec un ratio massique [oligosaccharide 2]/[insuline] de 2 et 100 mM de glutamate, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Glutamate de sodium | 1,691 g |

Pour le glutamate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B60. Préparation d'une solution d'insuline humaine à 100 UI/mL en présence d'oligosaccharide 2 et de 60 mM d'acide malique.

Pour un volume final de 100 mL de formulation avec un ratio massique [oligosaccharide 2]/[insuline] de 2 et 60 mM d'acide malique, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Acide malique | 0,805 g |

Pour l'acide malique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B61. Préparation d'une solution d'insuline humaine à 100 UI/mL en présence d'oligosaccharide 2 et de 80 mM de succinate.

Pour un volume final de 100 mL de formulation avec un ratio massique [oligosaccharide 2]/[insuline] de 2 et 80 mM de succinate, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Succinate de sodium | 1,296 g |

Pour le succinate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### BG2. Préparation d'une solution d'insuline humaine à 100 UI/mL en présence d'oligosaccharide 2 et de 50 mM d'adipate.

Pour un volume final de 100 mL de formulation avec un ratio massique [oligosaccharide 2]/[insuline] de 2 et 50 mM d'adipate, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Adipate de sodium | 0,951 g |

Pour l'adipate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### 863. : Préparation d'une solution d'insuline humaine à 100 UI/mL en présence d'oligosaccharide 2 et de 80 mM d'ascorbate.

Pour un volume final de 100 mL de formulation avec un ratio massique[oligosaccharide 2]/[insuline] de 2 et 80 mM d'ascorbate, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Ascorbate de sodium | 1,585 g |

Pour l'ascorbate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### 864. : Préparation d'une solution d'insuline humaine à 100 UI/mL en présence d'oligosaccharide 2 et de 10 mM d'oxalate,

Pour un volume final de 100 mL de formulation avec un ratio massique [oligosaccharide 2]/[insuline] de 2 et 10 mM d'oxalate, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Oxalate de sodium | 134 mg |

Pour l'oxalate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B65. : Préparation d'une solution d'insuline humaine à 100 UI/mL en présence d'oligosaccharide 2 et de 14,6 mg/mL d'acide polyglutamique.

Pour un volume final de 100 mL de formulation avec un ratio massique[oligosaccharide 2]/[acide polyglutamique]/[insuline] de 2/4/1, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Acide polyglutamique | 1460 mg |

Pour l'acide polyglutamique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### 866. : Préparation d'une solution d'insuline humaine à 100 UI/mL en présence d'oligosaccharide 2 et de 14,6 mg/mL d'acide polyaspartique.

Pour un volume final de 100 mL de formulation avec un ratio massique [oligosaccharide 2]/[acide polyaspartique]/[insuline] de 2/4/1, les différents réactifs sont mélangés en quantités précisées ci-dessous :

| | |
|---|---|
| Insuline humaine à 500 UI/mL | 20 mL |
| Solution d'oligosaccharide 2à 36,01 mg/mL | 20,27 mL |
| Solution 96,6 mM m-crésol/566 mM glycérine | 30 mL |
| Eau | 28,95 mL |
| Acide polyaspartique | 1460 mg |

Pour l'acide polyaspartique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B67. : Préparation d'une solution d'insuline humaine à 100 UI/mL en présence d'oligosaccharide 2 et de 14,6 mg/mL de composé polyanionique 2.

Pour un volume final de 100 mL de formulation avec un ratio massique [oligosaccharide 2]/[composé polyanionique 2]/[insuline] de 2/4/1, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Composé polyanionique 2 | 1460 mg |

Le composé polyanionique 2 peut être utilisé sous la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### 868. Préparation d'une solution d'insuline humaine à 100 UI/mL en présence d'oligosaccharide 2 et de 7,3 mg/mL de triphosphate.

Pour un volume final de 100 mL de formulation avec un ratio massique [oligosaccharide 2]/[triphosphate]/[insuline] de 2/2/1, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Triphosphate ou polyphosphate de sodium | 730 mg |

Pour le triophosphate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B69. Préparation d'une solution d'insuline humaine à 100 UI/mL en présence d'oligosaccharide 2 et de 14,6 mg/mL de poly(acide acrylique).

Pour un volume final de 100 mL de formulation avec un ratio massique [oligosaccharide 2]/[poly(acide acrylique)]/[insuline] de 2/4/1, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Poly(acide acrylique) | 1460 mg |

Pour le poly(acide acrylique), on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### 870. Préparation d'une solution d'insuline humaine à 100 UI/mL en présence d'oligosaccharide 2 et de 14,6 mg/mL d'alginate de sodium (bas poids moléculaire).

Pour un volume final de 100 mL de formulation avec un ratio massique [oligosaccharide 2]/[alginate]/[insuline] de 2/4/1, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Alginate | 1460 mg |

Pour l'alginate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B71. Préparation d'une solution d'insuline humaine à 100 UI/mL en présence d'oligosaccharide 2 et de 21,9 mg/mL de polymère à base d'acide glucuronique.

Pour un volume final de 100 mL de formulation avec un ratio massique [oligosaccharide 2]/[polymère à base d'acide glucuronique]/[insuline] de 2/6/1, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Polymère à base d'acide glucuronique | 2190 mg |

Pour le polymère à base d'acide glucuronique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B72. Préparation d'une solution d'insuline humaine à 100 UI/mL en présence d'oligosaccharide 2 et de 21,9 mg/mL de polymère à base d'acide galacturonique.

Pour un volume final de 100 mL de formulation avec un ratio massique [oligosaccharide 2]/[polymère à base d'acide galacturonique]/[insuline] de 2/6/1, les différents réactifs sont mélangés en quantités précisées ci-dessous :

| | |
|---|---|
| Insuline humaine à 500 UI/mL | 20 mL |
| Solution d'oligosaccharide 2 à 36,01 mg/mL | 20,27 mL |
| Solution 96,6 mM m-crésol/566 mM glycérine | 30 mL |
| Eau | 28,95 mL |
| Polymère à base d'acide galacturonique | 2190 mg |

Pour de polymère à base d'acide galacturonique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B73. Préparation d'une solution d'insuline humaine à 100 UI/mL en présence d'oligosaccharide 2 et de 21,9 mg/mL de polymère à base d'acide hyaluronique.

Pour un volume final de 100 mL de formulation avec un ratio massique [oligosaccharide 2]/[polymère à base d'acide hyaluronique]/[insuline] de 2/6/1, les différents réactifs sont mélangés en quantités précisées ci-dessous :

| | |
|---|---|
| Insuline humaine à 500 UI/mL | 20 mL |
| Solution d'oligosaccharide 2à 36,01 mg/mL | 20,27 mL |
| Solution 96,6 mM m-crésol/566 mM glycérine | 30 mL |
| Eau | 28,95 mL |
| Polymère à base d'acide hyaluronique | 2190 mg |

Pour de polymère à base d'acide hyaluronique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### 874, Préparation d'une solution d'insuline humaine à 100 UI/mL en présence d'oligosaccharide 1 et de 80 mM de tartrate.

Pour un volume final de 100 mL de formulation avec un ratio massique [oligosaccharide 1]/[insuline] de 2 et 80 mM de tartrate, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mM glycérine | 30 mL |
| Eau | 28,95 mL |
| Tartrate de sodium | 1,552 g |

Pour le tartarte, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B75. Préparation d'une solution d'insuline humaine à 100 UI/mL en présence d'oligosaccharide 1 et de 80 mM de phosphate.

Pour un volume final de 100 mL de formulation avec un ratio massique [oligosaccharide 1]/[insuline] de 2 et 80 mM de phosphate, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mMglycérine | 30 mL |
| Eau | 28,95 mL |
| Na₂HPO₄,12H₂O | 2,864 g |

Pour le phosphate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B76. : Préparation d'une solution d'insuline humaine à 100 UI/mL en présence d'oligosaccharide 1 et de 80 mM d'aspartate.

Pour un volume final de 100 mL de formulation avec un ratio massique [oligosaccharide 1]/[insuline] de 2 et 80 mM d'aspartate, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mM glycérine | 30 mL |
| Eau | 28,95 mL |
| Aspartate de sodium | 1,416 g |

Pour l'aspartate, on peut utiliser, la forme acide ou la forme basique sous forme de sel de sodium, de sel de potassium ou d'un autre sel compatible avec une formulation injectable,

Le pH final est de 7,4 ± 0,4.

Cette solution limpide est filtrée sur une membrane 0,22 µm puis est placée à +4°C.

### B77. Préparation d'une solution d'insuline humaine à 100 UI/mL en présence d'oligosaccharide 1 et de 100 mM de glutamate.

Pour un volume final de 100 mL de formulation avec un ratio massique [oligosaccharide 1]/[insuline] de 2 et 100 mM de glutamate, les différents réactifs sont mélangés en quantités précisées ci-dessous :

| | |
|---|---|
| Insuline humaine à 500 UI/mL | 20 mL |
| Solution d'oligosaccharide 1à 36,01 mg/mL | 20,27 mL |
| Solution 96,6 mM m-crésol/566 mM glycérine | 30 mL |
| Eau | 28,95 mL |
| Glutamate de sodium | 1,691 g |

Pour le glutamate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B78. Préparation d'une solution d'insuline humaine à 100 UI/mL en présence d'oligosaccharide 1 et de 60 mM d'acide malique.

Pour un volume final de 100 mL de formulation avec un ratio massique [oligosaccharide 2]/[insuline] de 1 et 60 mM d'acide malique, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mM glycérine | 30 mL |
| Eau | 28,95 mL |
| Acide malique | 0,805 g |

Pour l'acide malique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B79. Préparation d'une solution d'insuline humaine à 100 UI/mL en présence d'oligosaccharide 1 et de 80 mM de succinate,

Pour un volume final de 100 mL de formulation avec un ratio massique [oligosaccharide 1]/[insuline] de 2 et 80 mM de succinate, les différents réactifs sont mélangés en quantités précisées ci-dessous :

| | |
|---|---|
| Insuline humaine à 500 UI/mL | 20 mL |
| Solution d'oligosaccharide 1à 36,01 mg/mL | 20,27 mL |
| Solution 96,6 mM m-crésol/566 mM glycérine | 30 mL |
| Eau | 28,95 mL |
| Succinate de sodium | 1,296 g |

Pour le succinate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### 880. Préparation d'une solution d'insuline humaine à 100 UI/mL en présence d'oligosaccharide 1 et de 50 mM d'adipate.

Pour un volume final de 100 mL de formulation avec un ratio massique [oligosaccharide 1]/[insuline] de 2 et 50 mM d'adipate, les différents réactifs sont mélangés en quantités précisées ci-dessous :

| | |
|---|---|
| Insuline humaine à 500 UI/mL | 20 mL |
| Solution d'oligosaccharide 1à 36,01 mg/mL | 20,27 mL |
| Solution 96,6 mM m-crésol/566 mM glycérine | 30 mL |
| Eau | 28,95 mL |
| Adipate de sodium | 0,951 g |

Pour l'adipate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B81. Préparation d'une solution d'insuline humaine à 100 UI/mL en présence d'oligosaccharide 1 et de 80 mM d'ascorbate.

Pour un volume final de 100 mL de formulation avec un ratio massique [oligosaccharide 1]/[insuline] de 2 et 80 mM d'ascorbate, les différents réactifs sont mélangés en quantités précisées ci-dessous :

| | |
|---|---|
| Insuline humaine à 500 UI/mL | 20 mL |
| Solution d'oligosaccharide 1à 36,01 mg/mL | 20,27 mL |
| Solution 96,6 mM m-crésol/566 mM glycérine | 30 mL |
| Eau | 28,95 mL |
| Ascorbate de sodium | 1,585 g |

Pour l'ascorbate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### 882. Préparation d'une solution d'insuline humaine à 100 UI/mL en présence d'oligosaccharide 1 et de 10 mM d'oxalate

Pour un volume final de 100 mL de formulation avec un ratio massique [oligosaccharide 1]/[insuline] de 2 et 10 mM d'oxalate, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mM glycérine | 30 mL |
| Eau | 28,95 mL |
| Oxalate de sodium | 134 mg |

Pour l'oxalate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B83. Préparation d'une solution d'insuline humaine à 100 UI/mL en présence d'oligosaccharide 1 et de 14,6 mg/mL d'acide polyglutamique.

Pour un volume final de 100 mL de formulation avec un ratio massique [oligosaccharide 1]/[acide polyglutamique]/[insuline] de 2/4/1, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mM glycérine | 30 mL |
| Eau | 28,95 mL |
| Acide polyglutamique | 1460 mg |

Pour l'acide polyglutamique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B84. Préparation d'une solution d'insuline humaine à 100 UI/mL en présence d'oligosaccharide 1 et de 14,6 mg/mL d'acide polyaspartique.

Pour un volume final de 100 mL de formulation avec un ratio massique [oligosaccharide 1]/[acide polyaspartique]/[insuline] de 2/4/1, les différents réactifs sont mélangés en quantités précisées ci-dessous :

| | |
|---|---|
| Insuline humaine à 500 UI/mL | 20 mL |
| Solution d'oligosaccharide 1à 36,01 mg/mL | 20,27 mL |
| Solution 96,6 mM m-crésol/566 mM glycérine | 30 mL |
| Eau | 28,95 mL |
| Acide polyaspartique | 1460 mg |

Pour l'acide polyaspartique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B85. Préparation d'une solution d'insuline humaine à 100 UI/mL en présence d'oligosaccharide 1 et de 14,6 mg/mL de composé polyanionique 2.

Pour un volume final de 100 mL de formulation avec un ratio massique [oligosaccharide 1]/[composé polyanionique 2]/[insuline] de 2/4/1, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mM glycérine | 30 mL |
| Eau | 28,95 mL |
| Composé polyanionique 2 | 1460 mg |

Le composé polyanionique 2 peut être utilisé sous la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### 886. Préparation d'une solution d'insuline humaine à 100 UI/mL en présence d'oligosaccharide 1 et de 7,3 mg/mL de triphosphate.

Pour un volume final de 100 mL de formulation avec un ratio massique [oligosaccharide 1]/[triphosphate]/[insuline] de 2/2/1, les différents réactifs sont mélangés en quantités précisées ci-dessous :

| | |
|---|---|
| Insuline humaine à 500 UI/mL | 20 mL |
| Solution d'oligosaccharide 1à 36,01 mg/mL | 20,27 mL |
| Solution 96,6 mM m-crésol/566 mM glycérine | 30 mL |
| Eau | 28,95 mL |
| Triphosphate ou polyphosphate de sodium | 730 mg |

Pour le triophosphate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B87. Préparation d'une solution d'insuline humaine à 100 UI/mL en présence d'oligosaccharide 1 et de 14,6 mg/mL de poly(acide acrylique).

Pour un volume final de 100 mL de formulation avec un ratio massique [oligosaccharide 1]/[poly(acide acrylique)]/[insuline] de 2/4/1, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/565 mM glycérine | 30 mL |
| Eau | 28,95 mL |
| Poly(acide acrylique) | 1460 mg |

Pour le poly(acide acrylique), on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B88. Préparation d'une solution d'insuline humaine à 100 UI/mL en présence d'oligosaccharide 1 et de 14,6 mg/mL d'alginate de sodium (bas poids moléculaire).

Pour un volume final de 100 mL de formulation avec un ratio massique [oligosaccharide 1]/[alginate]/[insuline] de 2/4/1, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mM glycérine | 30 mL |
| Eau | 28,95 mL |
| Alginate | 1460 mg |

Pour l'alginate, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B89. Préparation d'une solution d'insuline humaine à 100 UI/mL en présence d'oligosaccharide 1 et de 21,9 mg/mL de polymère à base d'acide glucuronique,

Pour un volume final de 100 mL de formulation avec un ratio massique [oligosaccharide 1]/[polymère à base d'acide glucuronique]/[insuline] de 2/6/1, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mM glycérine | 30 mL |
| Eau | 28,95 mL |
| Polymère à base d'acide glucuronique | 2190 mg |

Pour le polymère à base d'acide glucuronique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B90. Préparation d'une solution d'insuline humaine à 100 UI/mL en présence d'oligosaccharide 1 et de 21,9 mg/mL de polymère à base d'acide galacturonique,

Pour un volume final de 100 mL de formulation avec un ratio massique [oligosaccharide 1]/[polymère à base d'acide galacturonique]/[insuline] de 2/6/1, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mM glycérine | 30 mL |
| Eau | 28,95 mL |
| Polymère à base d'acide galacturonique | 2190 mg |

Pour de polymère à base d'acide galacturonique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B91. Préparation d'une solution d'insuline humaine à 100 UI/mL en présence d'oligosaccharide 1 et de 21,9 mg/mL de polymère à base d'acide hyaluronique.

Pour un volume final de 100 mL de formulation avec un ratio massique [oligosaccharide 1]/[polymère à base d'acide hyaluronique]/[insuline] de 2/6/1, les différents réactifs sont mélangés en quantités précisées ci-dessous :

| | |
|---|---|
| Insuline humaine à 500 UI/mL | 20 mL |
| Solution d'oligosaccharide 1 à 36,01 mg/mL | 20,27 mL |
| Solution 96,6 mM m-crésol/566 mM glycérine | 30 mL |
| Eau | 28,95 mL |
| Polymère à base d'acide hyaluronique | 2190 mg |

Pour de polymère à base d'acide hyaluronique, on peut utiliser, la forme acide ou la forme basique sous forme de sel de sodium, de sel de potassium ou d'un autre sel compatible avec une formulation injectable.

Le pH final est de 7,4 ± 0,4.

Cette solution limpide est filtrée sur une membrane 0,22 µm puis est placée à +4°C.

### B92. Préparation d'une solution d'insuline humaine à 200 UI/mL.

60,4 g d'eau sont ajoutés à 884,7 mg d'insuline humaine comprenant 2 ion Zn2+ par hexamère, et le pH est ensuite ajusté de 5,7 à 3 par addition de 8 mL de solution 0,1 N d'HCl. La solution est neutralisée à pH 7 par addition 10 mL de solution 0,1 N de NaOH. La concentration est ensuite ajustée à 200 UI/mL avec 43,08 mL d'eau. Le pH final de cette solution est 7,02. La solution est enfin filtrée sur une membrane 0,22 µm.

### B93. Préparation d'une solution d'insuline humaine à 200 UI/mL en présence d'oligosaccharide 2 à 14,6 mg/mL et de 9,3 mM de citrate.

Pour un volume final de 100 mL de formulation avec un ratio massique (oligosaccharide 2/ insuline humaine) de 2, les différents réactifs sont mélangés en quantités précisées ci-dessous

| | |
|---|---|
| Insuline humaine à 200 UI/mL | 100 mL |
| Lyophilisat d'oligosaccharide 2 | 1460 mg |
| Solution de citrate de sodium à 1.188 M | 1570 µL |

Le pH final est ajusté à 7,4 ± 0,4.

La solution limpide est filtrée sur une membrane 0,22 µm et stockée à 4°C.

### B94. Préparation d'une solution d'insuline humaine à 200 UI/mL en présence d'oligosaccharide 2 à 14,6 mg/mL et de composé polyanionique 1 à 14,6 mg/mL.

Pour un volume final de 100 mL de formulation avec un ratio massique (oligosaccharide 2/composé polyanionique 1/insuline humaine) de 2/2/1, les différents réactifs sont mélangés en quantités précisées ci-dessous

| | |
|---|---|
| Insuline humaine à 200 UI/mL | 100 mL |
| Lyophilisat d'oligosaccharide 2 | 1460 mg |
| Lyophilisat de composé polyanionique 1 | 1460 mg |

Le pH final est ajusté à 7,4 ± 0,4.

La solution limpide est filtrée sur une membrane 0,22 µm et stockée à 4°C.

### 895. Préparation d'une solution d'insuline humaine à 200 UI/mL en présence d'oligosaccharide 2 à 14,6 mg/mL et de composé polyanionique 1 à 29,2 mg/mL.

Pour un volume final de 100 mL de formulation avec un ratio massique [oligosaccharide 2/composé polyanionique 1/insuline humaine] de 2/4/1, les différents réactifs sont mélangés en quantités précisées ci-dessous

| | |
|---|---|
| Insuline humaine à 200 UI/mL | 100 mL |
| Lyophilisat d'oligosaccharide 2 | 1460 mg |
| Lyophilisat de composé polyanionique 1 | 2920 mg |

Le pH final est ajusté à 7,4 ± 0,4.

La solution limpide est filtrée sur une membrane 0,22 µm et stockée à 4°C.

### B96. Préparation d'une solution d'insuline humaine à 100 UI/mL en présence d'oligosaccharide 3 à 7,3 mg/mL,

Pour un volume final de 100 mL de formulation avec un ratio massique [oligosaccharide 3/insuline humaine] de 2/1, les différents réactifs sont mélangés en quantités précisées ci-dessous

| | |
|---|---|
| Insuline humaine à 500 UI/mL | 20 mL |
| Oligosaccharide 3 à 27,71 mg/mL | 28,4 mL |
| 96,6 mM m-crésol/566 mM glycérol | 30 mL |
| Eau (volume pour dilution - volume d'hydroxyde de sodium) | 21,6 mL |

Le pH final est ajusté à 7,0 ± 0,3.

La solution limpide est filtrée sur une membrane 0,22 µm et stockée à 4°C.

Cette formulation est référencée exemple 18 dans le document de priorité.

### C PHARMACODYNAMIE ET PHARMACOCINETIQUE

### C1. Protocole de mesure de la Pharmacodynamie des solutions d'insuline,

12 porcs domestiques d'environ 50 kg, préalablement cathétérisés au niveau de la jugulaire, sont mis à jeun 2,5 heures avant le début de l'expérience. Dans l'heure précédant l'injection d'insuline, 3 prélèvements sanguins sont réalisés afin de déterminer le niveau basal de glucose.

L'injection d'insuline humaine à la dose de 0,125 IU/kg (ou de 0,09 UI/kg pour l'insuline analogue) est réalisée en sous-cutané au niveau du cou, sous l'oreille de l'animal à l'aide du stylo à insuline Novopen équipé d'une aiguille 31 G.

Des prélèvements sanguins sont ensuite réalisés toutes les 4 minutes pendant 20 min puis toutes les 10 minutes jusqu'à 3 heures. Après chaque prélèvement, le cathéter est rincé avec une solution diluée d'héparine.

Une goutte de sang est prélevée pour déterminer la glycémie au moyen d'un glucomètre.

Les courbes de pharmacodynamie du glucose sont ensuite tracées et le temps nécessaire pour atteindre le taux minimum de glucose dans le sang pour chaque porc est déterminé et reporté comme Tmin glucose. La moyenne des Tmin glucose est ensuite calculée.

Le sang restant est collecté dans un tube sec et centrifugé pour isoler le sérum. Les taux d'insuline dans les échantillons de sérum sont mesurés par la méthode immuno-enzymatique Elisa Sandwich pour chaque porc.

Les courbes de pharmacocinétique sont ensuite tracées. Le temps nécessaire pour atteindre la concentration maximale d'insuline dans le sérum pour chaque porc est déterminé et reporté comme Tmax insuline. La moyenne des Tmax insuline est ensuite calculée.

### C2. Résultats de Pharmacodynamie et de Pharmacocinétique des solutions d'insulines des exemples B1 et B3.

| Exemple | Insuline | Oligosaccharide | Excipient | Dose (IU/kg) | Nombre de porcs |
|---|---|---|---|---|---|
| B1 | Aspart | - | - | 0,125 | 11 |
| B3 | Humaine | - | - | 0,125 | 11 |

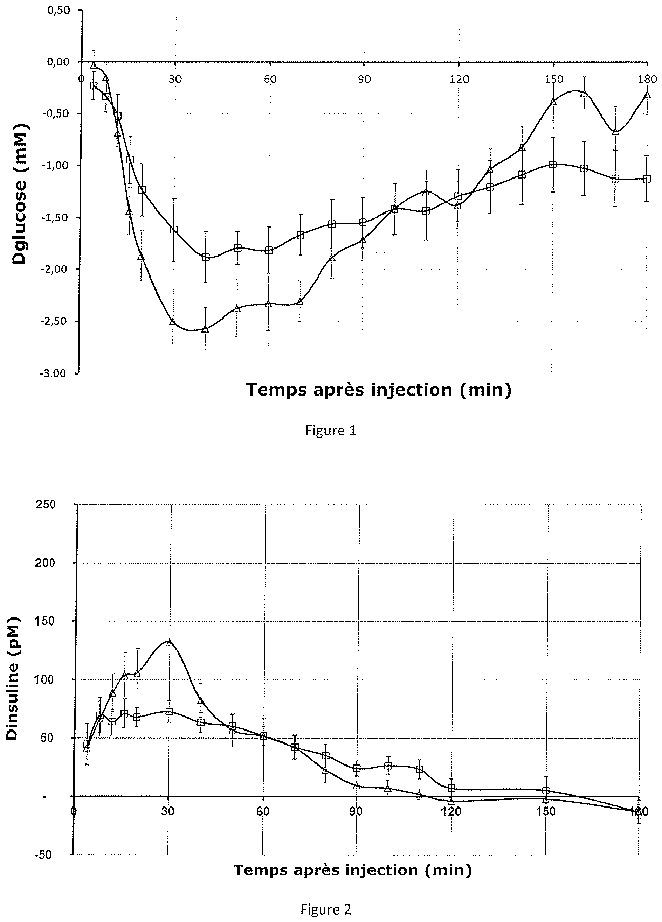

Les résultats de pharmacodynamie obtenus avec les formulations décrites dans les exemples B1 et B3 sont présentés sur la figure 1. L'analyse de ces courbes montre que la formulation d'insuline humaine (courbe tracée avec les carrés correspondant à l'exemple B3, Tmin glucose = 61 ± 31 min) a bien une action plus lente celle de de la formulation commerciale d'insuline aspart (courbe tracée avec les triangles correspondant à l'exemple B1, Tmin glucose = 44 ± 13 min).

Les résultats de pharmacocinétique obtenus avec les formulations décrites dans les exemples 81 et B3 sont présentés sur la figure 2. L'analyse de ces courbes montre que la formulation d'insuline humaine seule (courbe tracée avec les carrés correspondant à l'exemple B3, Tmax insuline = 36 ± 33 min) induit bien une absorption plus lente que la formulation commerciale d'insuline aspart (Novolog^{®}) (courbe tracée avec les triangles correspondant à l'exemple B1, Tmax insuline = 28 ± 13 min). Ces résultats sont conformes à ceux de la littérature avec une accélération d'un analogue rapide d'insuline par rapport à une insuline humaine et valident donc l'adéquation du modèle à la problématique de la mesure de l'accélération d'une insuline.

### C3. Résultats de Pharmacodynamie et de Pharmacocinétique des solutions d'insulines des exemples B1 et B6.

| Exemple | Insuline | Oligosaccharide | Excipient | Dose IU/kg | Nombre de porcs |
|---|---|---|---|---|---|
| B1 | Aspart | - | - | 0,125 | 16 |
| B6 | Humaine | 2 | Composé polyanionique 1 7.3 mg/mL | 0,125 | 8 |

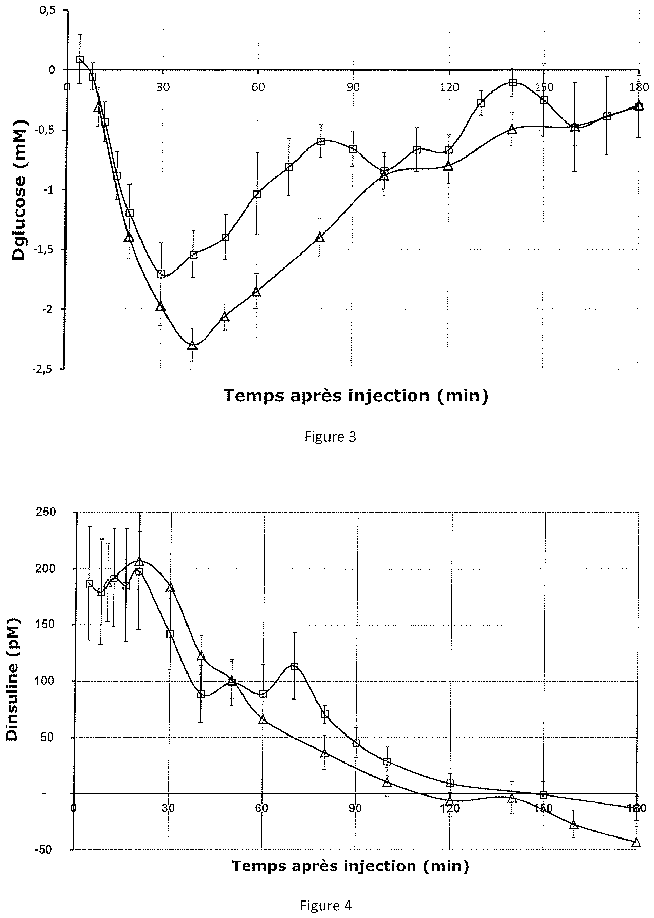

Les résultats de pharmacodynamie obtenus avec les formulations décrites dans les exemples B1 et B6 sont présentés sur la figure 3. L'analyse de ces courbes montre que la formulation à base d'insuline humaine comprenant l'oligosaccharide 2 et le composé polyanionique 1 comme excipient à 7,3 mg/mL (courbe tracée avec les carrés correspondant à l'exemple B6, Tmin glucose = 39 ± 11 min) permet d'obtenir une action aussi rapide que celle de de la formulation commerciale d'insuline aspart (Novolog^{®}) (courbe tracée avec les triangles correspondant à l'exemple B1, Tmin glucose = 41 ± 9 min).

Les résultats de pharmacocinétique obtenus avec les formulations décrites dans les exemples B1 et B6 sont présentés sur la figure 4. L'analyse de ces courbes montre que la formulation à base d'insuline humaine comprenant l'oligosaccharide 2 et le composé polyanionique 1 comme excipients à 7,3 mg/mL (courbe tracée avec les carrés correspondant à l'exemple B6 , Tmax insuline = 14 ± 9 min) induit une absorption plus rapide que la formulation commerciale d'insuline aspart (Novolog^{®}) (courbe tracée avec les triangles correspondant à l'exemple B1, Tmax insuline = 24 ± 13 min). Les paramètres de temps de l'insuline aspart entre les exemples C2 et C3 étant similaires, on peut en déduire par extrapolation que la formulation de l'exemple B6 induit aussi une accélération par rapport à l'insuline humaine (exemple B3).

### C4. Résultats de Pharmacodynamie et de Pharmacocinétique des solutions d'insulines des exemples B1 et B7

| Exemple | Insuline | Oligosaccharide | Excipient | Dose IU/kg | Nombre de porcs |
|---|---|---|---|---|---|
| B1 | Aspart | - | - | 0,125 | 14 |
| B7 | Humaine | 2 | Citrate 9,3 mM | 0,125 | 12 |

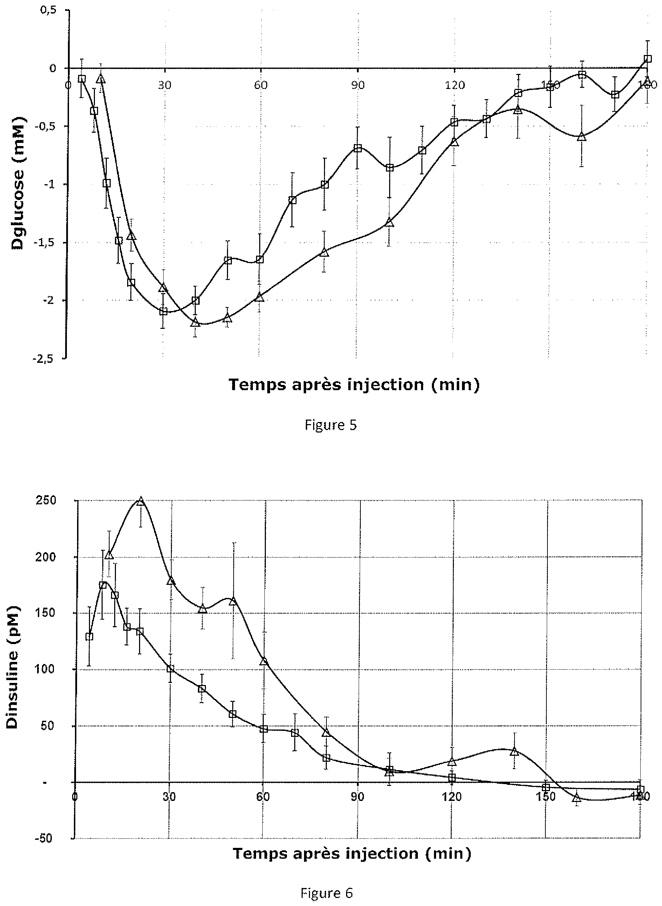

Les résultats de pharmacodynamie obtenus avec les formulations décrites dans les exemples 81 et B7 sont présentés sur la figure 5. L'analyse de ces courbes montre que la formulation à base d'insuline humaine comprenant l'oligosaccharide 2 et le citrate à 9,3 mM comme excipients (courbe tracée avec les carrés correspondant à l'exemple B7, Tmin glucose = 36 ± 14 min) permet d'obtenir une action plus rapide que celle de de la formulation commerciale d'insuline aspart (Novolog^{®}) (courbe tracée avec les triangles correspondant à l'exemple B1, Tmin glucose = 53 ± 24 min).

Les résultats de pharmacocinétique obtenus avec les formulations décrites dans les exemples B1 et B7 sont présentés sur la figure 6. L'analyse de ces courbes montre que la formulation comprenant l'oligosaccharide 2 et le citrate à 9,3 mM comme excipients (courbe tracée avec les carrés correspondant à l'exemple B7, Tmax insuline = 15 ± 10 min) induit une absorption plus rapide que la formulation commerciale d'insuline aspart (Novolog^{®}) (courbe tracée avec les triangles correspondant à l'exemple B1, Tmax insuline = 22 ± 10 min).Les paramètres de temps de l'insuline aspart (Novolog^{®}) entre les exemples C2 et C4 étant similaires, on peut en déduire par extrapolation que la formulation de l'exemple B7 induit aussi une accélération par rapport à l'insuline humaine (exemple B3).

### C5. Résultats de Pharmacodynamie et de Pharmacocinétique des solutions d'insulines des exemples B2 et B9

| Exemple | Insuline | Oligosaccharide | Excipient | Dose IU/kq | Nombre de porcs |
|---|---|---|---|---|---|
| B2 | Lispro | - | - | 0,09 | 23 |
| 89 | Lispro | 2 | Citrate 9,3 mM | 0,09 | 12 |

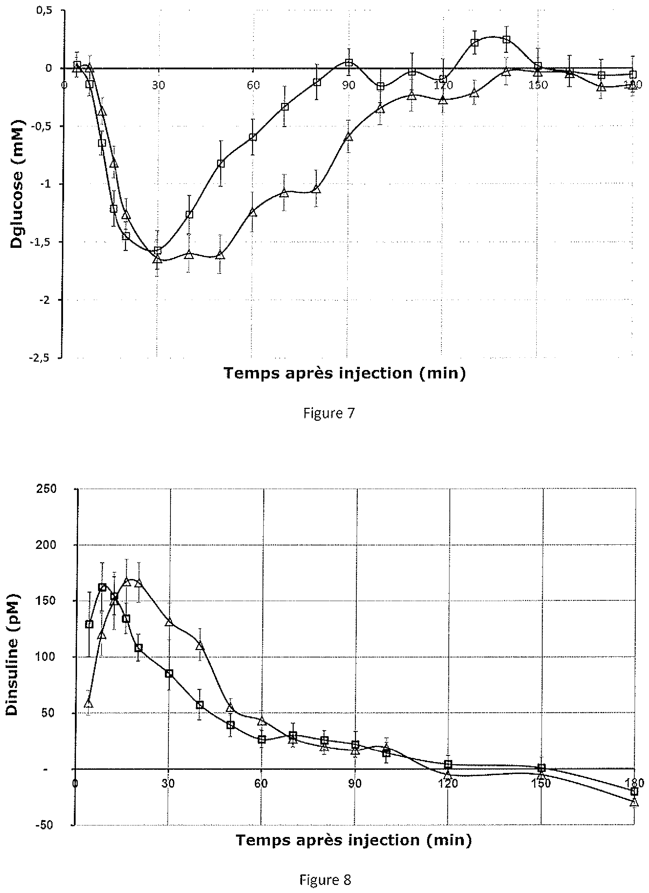

Les résultats de pharmacodynamie obtenus avec les formulations décrites dans les exemples B2 et B9 sont présentés sur la figure 7. L'analyse de ces courbes montre que la formulation à base d'insuline lispro (Humalog^{®}) comprenant l'oligosaccharide 2 et le citrate à 9,3mM comme excipients (courbe tracée avec les carrés correspondant à l'exemple B9, Tmin glucose = 32 ± 9 min) permet d'obtenir une action plus rapide que celle de de la formulation commercialed'insuline lispro (Humalog^{®}) (courbe tracée avec les triangles correspondant à l'exemple B2, Tmin glucose = 45 ± 16 min).

Les résultats de pharmacocinétique obtenus avec les formulations décrites dans les exemples B2 et B9 sont présentés sur la figure 8. Selon l'invention, l'analyse de ces courbes montrent que la formulation à base de Humalog^{®} comprenant l'oligosaccharide 2 et le citrate à 9.3mM comme excipient (courbe tracée avec les carrés correspondant à l'exemple B9, Tmax insuline = 12 ± 7 min) induit une absorption plus rapide que la formulation commerciale d'insuline lispro (Humalog^{®}) (courbe tracée avec les triangles correspondant à l'exemple B2, Tmax insuline = 19 ± 10 min).

### C6. Résultats de Pharmacodynamie et de Pharmacocinétique des solutions d'insulines des exemples B2 et B10

| Exemple | Insuline | Oligosaccharide | Excipient | Dose IU/kg | Nombre de porcs |
|---|---|---|---|---|---|
| B2 | Lispro | - | - | 0,09 | 11 |
| B10 | Lispro | 2 | Citrate 6 mM | 0,09 | 10 |

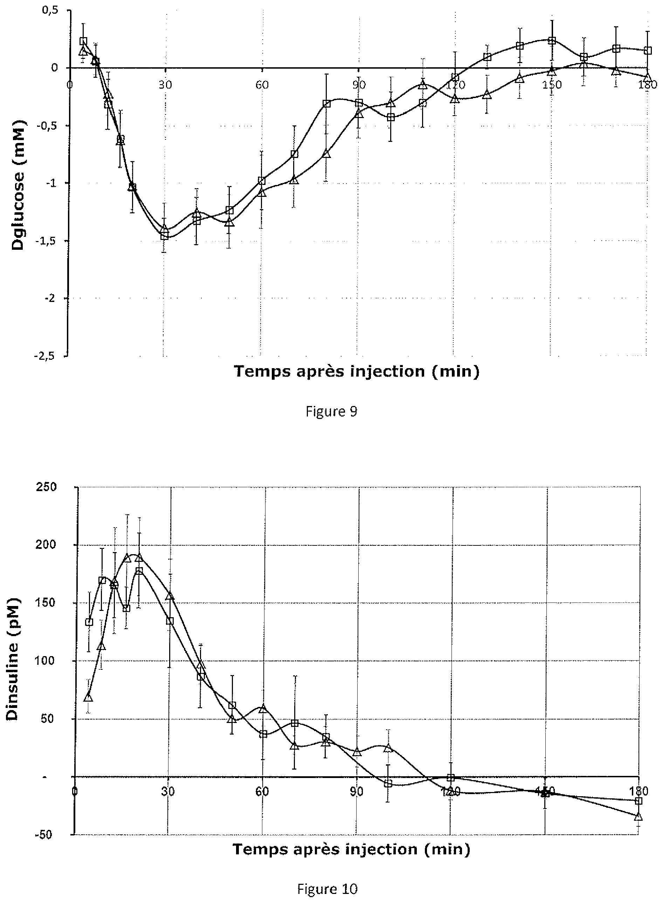

Les résultats de pharmacodynamie obtenus avec les formulations décrites dans les exemples B2 et 810 sont présentés sur la figure 9. L'analyse de ces courbes montre que la formulation à base d'insuline lispro (Humalog^{®}) comprenant l'oligosaccharide 2 et le citrate à 6 mM comme excipients (courbe tracée avec les carrés correspondant à l'exemple 810, Tmin glucose = 34 ± 12 min) permet d'obtenir une action plus rapide que celle de de la formulation commercialed'insuline lispro (Humalog^{®}) (courbe tracée avec les triangles correspondant à l'exemple B2, Tmin glucose = 44 ± 14 min).

Les résultats de pharmacocinétique obtenus avec les formulations décrites dans les exemples B2 et B10 sont présentés sur la figure 10. L'analyse de ces courbes montre que la formulation à base d'insuline lispro (Humalog^{®}) comprenant l'oligosaccharide 2 et le citrate à 6 mM comme excipients (courbe tracée avec les carrés correspondant à l'exemple B10, Tmax insuline = 13 ± 8 min) induit une absorption plus rapide que la formulation commerciale d'insuline lispro (Humalog^{®}) (courbe tracée avec les triangles correspondant à l'exemple B2, Tmax insuline = 18 ± 8 min).

### C7. Résultats de Pharmacodynamie et de Pharmacocinétique des solutions d'insulines des exemples B2 et B11.

| Exemple | Insuline | Oligosaccharide | Excipient | Dose IU/kg | Nombre de porcs |
|---|---|---|---|---|---|
| B2 | Lispro | - | - | 0,09 | 11 |
| 811 | Lispro | 1 | Citrate 9,3 mM | 0,09 | 11 |

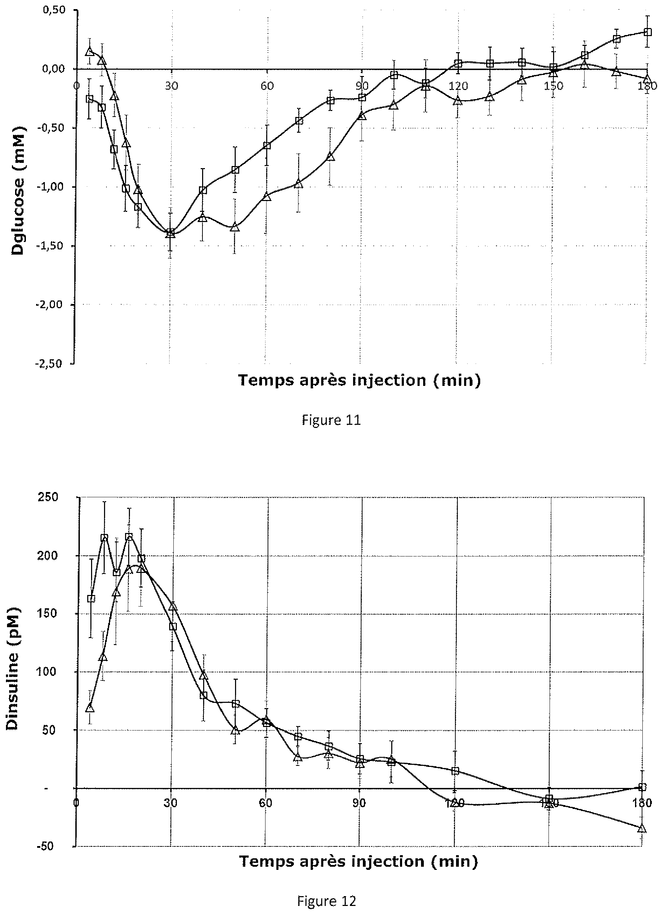

Les résultats de pharmacodynamie obtenus avec les formulations décrites dans les exemples B2 et B11 sont présentés sur la figure 11. L'analyse de ces courbes montre que la formulation à base d'insuline lispro (Humalog^{®}) comprenant l'oligosaccharide 1 et le citrate à 9,3 mM comme excipients (courbe tracée avec les carrés correspondant à l'exemple 811, Tmin glucose = 31 ± 14 min) permet d'obtenir une action plus rapide que celle de la formulation commercialed'insuline lispro (Humalog^{®}) (courbe tracée avec les triangles correspondant à l'exemple B2, Tmin glucose = 44 ± 14 min).

Les résultats de pharmacocinétique obtenus avec les formulations décrites dans les exemples B2 et B11 sont présentés sur la figure 12. L'analyse de ces courbes montre que la formulation à base d'insuline lispro(Humalog^{®}) comprenant l'oligosaccharide 1 et le citrate à 9,3 mM comme excipients (courbe tracée avec les carrés correspondant à l'exemple B11, Tmax insuline = 15 ± 7 min) induit une absorption plus rapide que la formulation commerciale d'insulilne lispro (Humalog^{®}) (courbe tracée avec les triangles correspondant à l'exemple B2, Tmax insuline = 18 ± 8 min).

### C8. Résultats de Pharmacodynamie et de Pharmacocinétique des solutions d'insulines des exemples B2 et B12.

| Exemple | Insuline | Oligosaccharide | Excipient | Dose IU/kg | Nombre de porcs |
|---|---|---|---|---|---|
| B2 | Lispro | - | - | | 9 |
| B12 | Lispro | 1 | Citrate 18,6 mM | 0,09 | 11 |

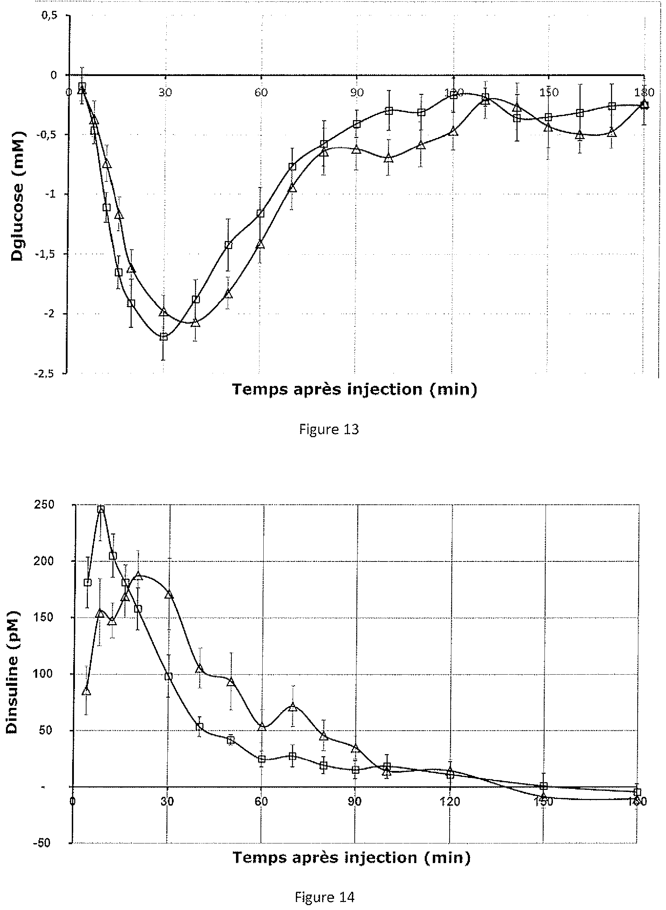

Les résultats de pharmacodynamie obtenus avec les formulations décrites dans les exemples B2 et B12 sont présentés sur la figure 13. L'analyse de ces courbes montre que la formulation à base d'insuline lispro (Humalog^{®}) comprenant l'oligosaccharide 1 et le citrate à 18,6 mM comme excipients (courbe tracée avec les carrés correspondant à l'exemple B12, Tmin glucose = 30 ± 5 min) permet d'obtenir une action plus rapide que celle de de la formulation commercialed'insuline lispro (Humalog^{®}) (courbe tracée avec les triangles correspondant à l'exemple B2, Tmin glucose = 40 ± 12 min).

Les résultats de pharmacocinétique obtenus avec les formulations décrites dans les exemples B2 et B12 sont présentés sur la figure 14. L'analyse de ces courbes montre que la formulation à base d'insuline lispro (Humalog^{®}) comprenant l'oligosaccharide 1 et le citrate à 18,6 mM comme excipients (courbe tracée avec les carrés correspondant à l'exemple B11, Tmax insuline = 10 ± 4 min) induit une absorption plus rapide que la formulation commerciale d'insuline lispro (Humalog^{®}) (courbe tracée avec les triangles correspondant à l'exemple B2, Tmax insuline = 23 ± 12 min).

### C9. Résultats de Pharmacodynamie et de Pharmacocinétique des solutions d'insulines des exemples B2 et B13.

| Exemple | Insuline | Oligosaccharide | Excipient | Dose IU/kg | Nombre de porcs |
|---|---|---|---|---|---|
| B2 | Lispro | - | - | 0,09 | 9 |
| B13 | Lispro | 2 | Composé polyanionique 1 7 , 3 mg/mL | 0,09 | 11 |

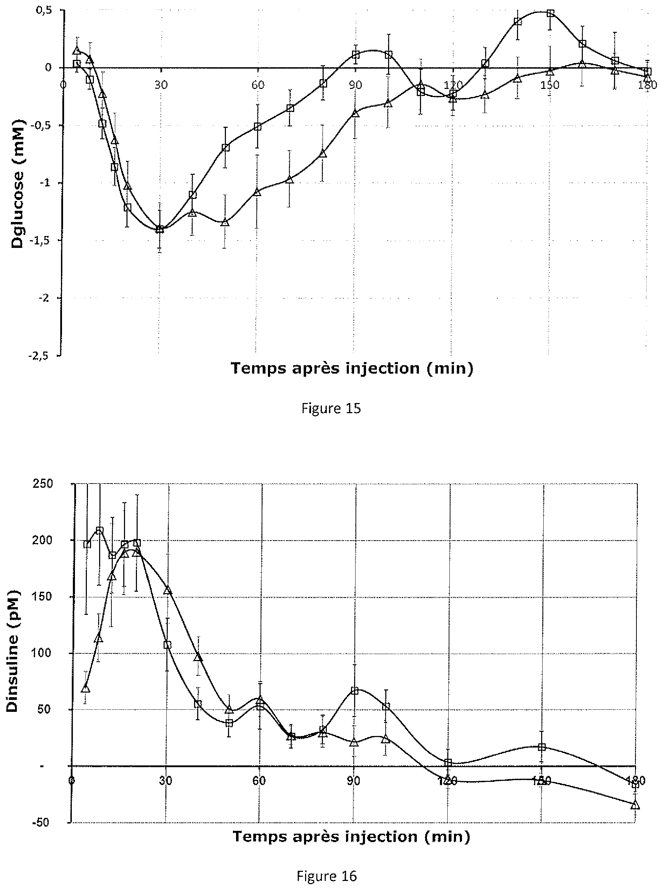

Les résultats de pharmacodynamie obtenus avec les formulations décrites dans les exemples B2 et B13 sont présentés sur la figure 15. L'analyse de ces courbes montre que la formulation à base d'insuline lispro (Humalog^{®}) comprenant l'oligosaccharide 2 et le composé polyanionique 1 comme excipients à 7,3 mg/mL (courbe tracée avec les carrés correspondant à l'exemple B13, Tmin glucose = 32 ± 12 min) permet d'obtenir une action plus rapide que celle de de la formulation commercialed'insuline lispro (Humalog^{®}) (courbe tracée avec les triangles correspondant à l'exemple B2, Tmin glucose = 44 ± 14 min).

Les résultats de pharmacocinétique obtenus avec les formulations décrites dans les exemples B2 et B13 sont présentés sur figure 16. L'analyse de ces courbes montre que la formulation à based'insuline lispro (Humalog^{®})comprenant l'oligosaccharide 2 et le composé polyanionique 1 comme excipients à 7,3 mg/mL (courbe tracée avec les carrés correspondant à l'exemple B13, Tmax insuline = 14 ± 7 min) induit une absorption plus rapide que la formulation commercialed'insuline lispro Humalog^{®}) (courbe tracée avec les triangles correspondant à l'exemple B2, Tmax insuline = 18 ± 8 min).

### C10. Résultats de Pharmacodynamie et de Pharmacocinétique des solutions d'insulines des exemples B2 et B14.

| Exemple | Insuline | Oligosaccharide | Excipient | Dose IU/kg | Nombre de porcs |
|---|---|---|---|---|---|
| B2 | Lispro | - | - | 0,09 | 10 |
| B14 | Lispro | 2 | Composé polyanionique 1 14,6 mg/mL | 0,09 | 11 |

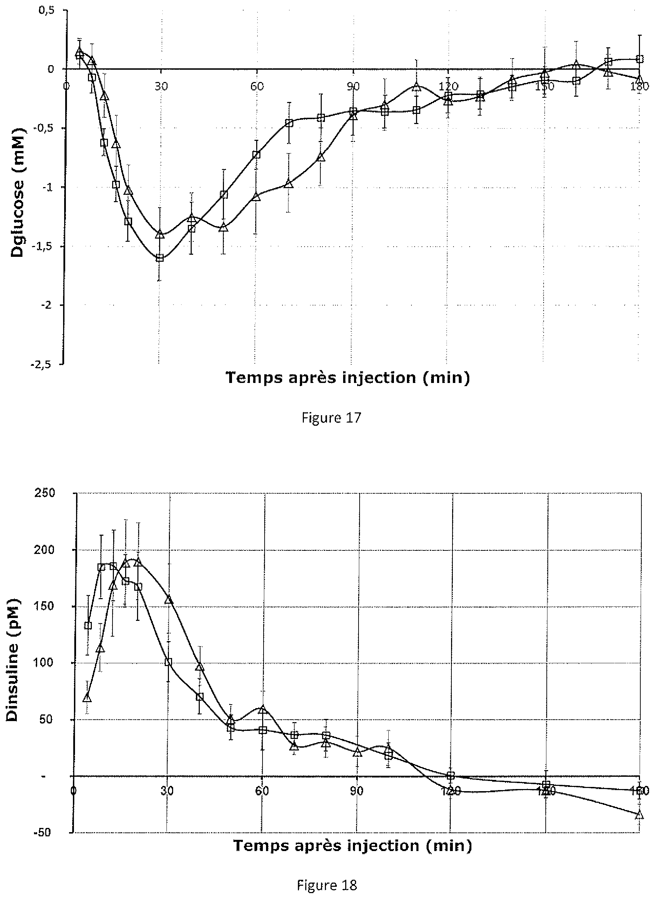

Les résultats de pharmacodynamie obtenus avec les formulations décrites dans les exemples B2 et B14 sont présentés sur la figure 17. L'analyse de ces courbes montre que la formulation à base d'insuline lispro(Humalog^{®}) comprenant l'oligosaccharide 2 et le composé polyanionique 1 comme excipient à 14,6 mg/mL (courbe tracée avec les carrés correspondant à l'exemple B14, Tmin glucose = 30 ± 7 min) permet d'obtenir une action plus rapide que celle de de la formulation commercialed'insuline lispro (Humalog^{®}) (courbe tracée avec les triangles correspondant à l'exemple B2, Tmin glucose = 44 ± 14 min).

Les résultats de pharmacocinétique obtenus avec les formulations décrites dans les exemples B2 et B14 sont présentés sur la figure 18. L'analyse de ces courbes montre que la formulation à base d'insuline lispro (Humalog^{®}) comprenant l'oligosaccharide 2 et le composé polyanionique 1 comme excipient à 14,6 mg/mL (courbe tracée avec les carrés correspondant à l'exemple B14, Tmax insuline = 12 ± 5 min) induit une absorption plus rapide de Humalog^{®} que la formulation commercialed'insuline lispro (Humalog^{®}) (courbe tracée avec les triangles correspondant à l'exemple B2, Tmax insuline = 18 ± 8 min).

### C11. Résultats de Pharmacodynamie et de Pharmacocinétique des solutions d'insulines des exemples B2 et B8.

| Exemple | Insuline | Oligosaccharide | Excipient | Dose IU/kg | Nombre de porcs |
|---|---|---|---|---|---|
| B2 | Lispro | - | - | 0,09 | 12 |
| B8 | Lispro | 6 | - | 0,09 | 12 |

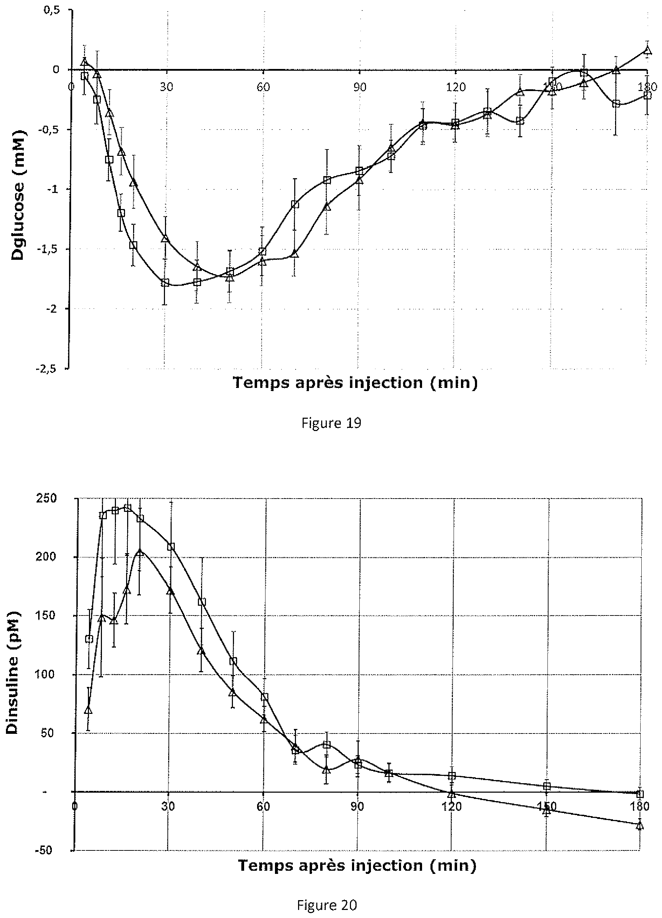

Les résultats de pharmacodynamie obtenus avec les formulations décrites dans les exemples B2 et B8 sont présentés sur la figure 19. L'analyse de ces courbes montre que la formulation à base d'insuline lispro(Humalog^{®}) comprenant l'oligosaccharide 6 (courbe tracée avec les carrés correspondant à l'exemple B8, Tmin glucose = 45 ± 19 min) ne permet pas d'obtenir une action plus rapide que celle de de la formulation commercialed'insuline lispro (Humalog^{®}) (courbe tracée avec les triangles correspondant à l'exemple B2, Tmin glucose = 50 ± 14 min).

Les résultats de pharmacocinétique obtenus avec les formulations décrites dans les exemples B2 et B8 sont présentés sur la figure 20. L'analyse de ces courbes montre que la formulation à base d'insuline lispro (Humalog^{®}) comprenant l'oligosaccharide 6 (courbe tracée avec les carrés correspondant à l'exemple B8, Tmax insuline = 18 ± 10 min) n'induit pas une absorption plus rapide de Humalog^{®} que la formulation commercialed'insuline lispro (Humalog^{®}) (courbe tracée avec les triangles correspondant à l'exemple B2, Tmax insuline = 20 ± 9 min).

### C12. Résultats de Pharmacodynamie et de Pharmacocinétique des solutions d'insulines des exemples B9 et B8.

| Exemple | Insuline | Oligosaccharide | Excipient | Dose IU/kg | Nombre de porcs |
|---|---|---|---|---|---|
| B9 | Lispro | 2 | Citrate 9,3mM | 0,09 | 12 |
| B8 | Lispro | 6 | - | 0,09 | 12 |

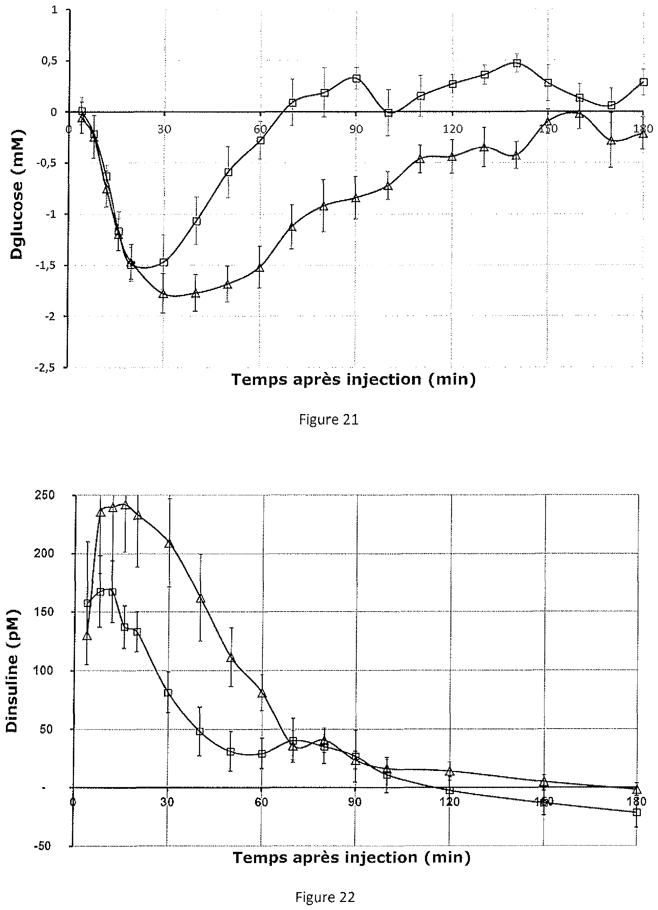

Les résultats de pharmacodynamie obtenus avec les formulations décrites dans les exemples B8 et B9 sont présentés sur la figure 21. L'analyse de ces courbes montre que la formulation à base d'insuline lispro (Humalog^{®}) comprenant l'oligosaccharide 2 et le citrate à 9,3 mM comme excipient (courbe tracée avec les carrés correspondant à l'exemple B9, Tmin glucose = 32 ± 9 min) permet d'obtenir une action plus rapide que celle de la formulation à base d'insuline lispro (Humalog^{®}) comprenant l'oligosaccharide 6 (courbe tracée avec les triangles correspondant à l'exemple B8, Tmin glucose = 45 ± 19 min).

Les résultats de pharmacocinétique obtenus avec les formulations décrites dans les exemples B8 et B9 sont présentés sur la figure 22. Selon l'invention, l'analyse de ces courbes montrent que la formulation à base de Humalog^{®} comprenant l'oligosaccharide 2 et le citrate à 9.3mM comme excipient (courbe tracée avec les carrés correspondant à l'exemple B9, Tmax insuline = 12 ± 7 min) induit une absorption plus rapide que la formulation à base d'insuline lispro (Humalog^{®}) comprenant l'oligosaccharide 6 (courbe tracée avec les triangles correspondant à l'exemple B8, Tmax insuline = 18 ± 10min).

### C13. Résultats de Pharmacodynamie et de Pharmacocinétique des solutions d'insulines des exemples B2 et B15.

| Exemple | Insuline | Oligosaccharide | Excipient | Dose IU/kg | Nombre de porcs |
|---|---|---|---|---|---|
| B2 | Lispro | - | - | 0,09 | 12 |
| B15 | Lispro | 2 à 14,3 mg/mL | - | 0,09 | 12 |

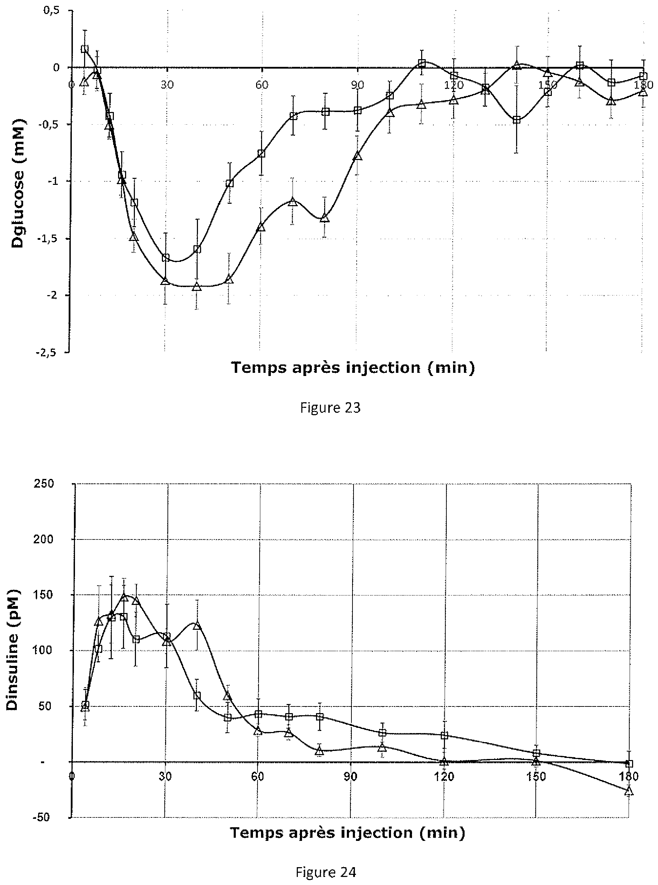

Les résultats de pharmacodynamie obtenus avec les formulations décrites dans les exemples B2 et B15 sont présentés sur la figure 23. L'analyse de ces courbes montre que la formulation à base d'insuline lispro(Humalog^{®}) comprenant l'oligosaccharide 2 à 14,6 mg/mL (courbe tracée avec les carrés correspondant à l'exemple B15, Tmin glucose =35 ± 5 min) permet d'obtenir une action plus rapide que celle de de la formulation commercialed'insuline lispro (Humalog^{®}) (courbe tracée avec les triangles correspondant à l'exemple B2, Tmin glucose = 47 ± 18 min).

Les résultats de pharmacocinétique obtenus avec les formulations décrites dans les exemples B2 et B15 sont présentés sur la figure 24. L'analyse de ces courbes montre que la formulation à base d'insuline lispro (Humalog^{®}) comprenant l'oligosaccharide 2 à 14,6 mg/Ml (courbe tracée avec les carrés correspondant à l'exemple B15, Tmax insuline = 12 ± 4 min) induit une absorption plus rapide de Humalog^{®} que la formulation commercialed'insuline lispro (Humalog^{®}) (courbe tracée avec les triangles correspondant à l'exemple B2, Tmax insuline =20 ± 11 min).

### C14. Résultats de Pharmacodynamie et de Pharmacocinétique des solutions d'insulines des exemples B3 et B96

| Exemple | Insuline | Oligosaccharide | Excipient | Dose IU/kg | Nombre de porcs |
|---|---|---|---|---|---|
| B3 | Humaine | - | - | 0,125 | 10 |
| B96 | Humaine | 3 | - | 0,125 | 9 |

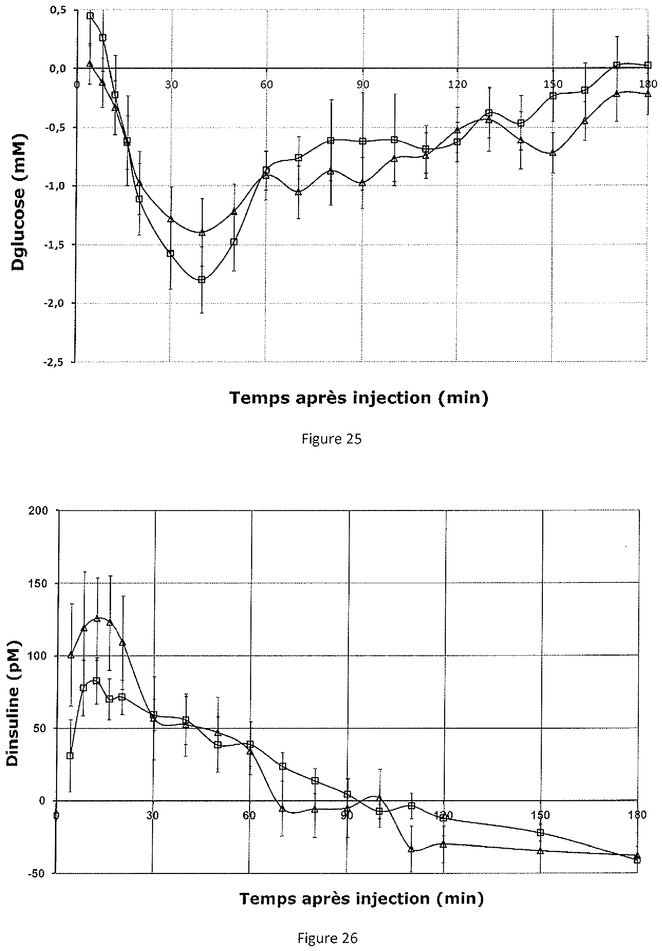

Les résultats de pharmacodynamie obtenus avec les formulations décrites dans les exemples B3 et B96 sont présentés sur la figure 25. L'analyse de ces courbes montre que la formulation à base d'insuline humaine comprenant l'oligosaccharide 3 comme excipient à 7,3 mg/mL (courbe tracée avec les carrés correspondant à l'exemple B96, Tmin glucose = 46±20 min) permet d'obtenir une action plus rapide que celle de de la formulation commerciale d'insuline humaine (courbe tracée avec les triangles correspondant à l'exemple B3, Tmin glucose = 64 ± 33 min).

Les résultats de pharmacocinétique obtenus avec les formulations décrites dans les exemples B3 et B96 sont présentés sur la figure 26. L'analyse de ces courbes montre que la formulation à base d'insuline humaine comprenant l'oligosaccharide 3 comme excipient à 7,3 mg/mL (courbe tracée avec les carrés correspondant à l'exemple B96 , Tmax insuline = 12 ± 6 min) induit une absorption plus rapide que la formulation commerciale d'insuline humaine (courbe tracée avec les triangles correspondant à l'exemple B3, Tmax insuline = 26 ± 20 min).

### D Dichroïsme circulaire

### D1. Etat d'association de l'insuline lispro (Humalog^{®}) par dichroïsme circulaire (CD) en présence des oligosaccharides

Le dichroïsme circulaire permet d'étudier la structure secondaire et quaternaire de l'insuline. Les monomères d'insuline s'organisent en dimères et en hexamères. L'hexamère est la forme de l'insuline la plus stable physiquement et chimiquement. Il existe deux formes hexamèriques, la forme R6 et la forme T6. L'insuline lispro présente un signal CD fort à 251nm caractéristique de la forme hexamèrique R6 (forme la plus stable). La perte du signal CD à 251nm est relié à une déstabilisation de l'hexamère (et donc le premier signe de transformation de l'hexamère en dimère).

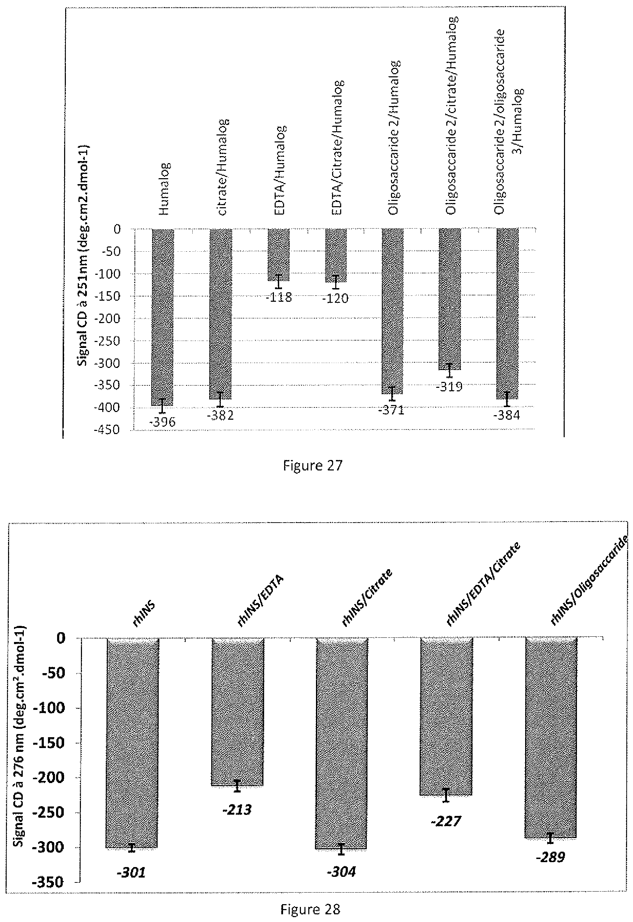

L'EDTA et le mélange EDTA/citrate déstructure complètement la forme R6 de l'insuline lispro(figure 27). L'EDTA a donc un effet marqué de déstabilisation de l'hexamère. Au contraire, le citrate seul, l'oligosaccharide 2 seul ainsi que le mélange oligosaccharide 2/citrate n'a quasiment pas d'impact sur le signal CD à 251nm. Ces composés n'ont donc quasiment aucun impact sur la structure R6 de l'hexamère et à fortiori sur la structure hexamèrique de l'insuline, à la différence de l'EDTA qui déstabilisé l'hexamère,

### D2, Etat d'association de l'insuline humaine par dichroïsme circulaire (CD) en présence des oligosaccharides

Le signal CD à 276nm (en absence de m-crésol) est caractéristique de la forme hexamèrique de l'insuline humaine (signal de l'hexamère aux alentours de -300 nm, signal du dimère entre -200 nm et -250 nm et signal du monomère en-dessous de - 200). La perte du signal CD à 276 nm est donc caractéristique d'une déstabilisation de l'hexamère en dimères ou monomères.

L'EDTA et la combinaison EDTA/citrate ont un impact très marqué sur la structure hexamèrique de l'insuline humaine (dissociation complète de l'hexamère en dimères, figure 28). Au contraire, l'oligosaccharide 1 n'a pas d'impact significatif sur la structure hexamèrique de l'insuline humaine. Contrairement à l'EDTA, les formulations à base d'oligosaccharide 1 ne dissocient pas l'hexamère de l'insuline humaine.

### E Solubilisation d'insuline humaine et analogue au point isoélectrique

### E1. Solubilisation de l'insuline humaine à son point isoélectrique

L'insuline humaine a un point isoélectrique à 5,3. A ce pH l'insuline humaine précipite. Un test démontrant la formation d'un complexe de l'insuline humaine avec les différents oligosaccharides ou polysaccharides est exécuté au point isoélectrique. Si une interaction existe, il est possible de solubiliser l'insuline à son point isoélectrique.

Une solution d'insuline humaine à 200 UI/mL est préparée. Des solutions d'oligosaccharides ou de polysaccharides à différentes concentrations (8, 30 ou 100 mg/mL) dans l'eau sont préparées. Un mélange équivolume (50/50) entre la solution d'insuline et la solution d'oligosaccharide ou de polysaccharide est effectué pour mener à une solution contenant 100 UI/ML d'insuline humaine et la concentration désirée de polysaccharide (4, 15 ou 50 mg/mL). Le pH des différentes solutions est ajusté à pH 5,3 par ajout d'acide acétique 200 mM.

L'aspect de la solution est documenté. Si la solution est turbide, l'oligosaccharide ou le polysaccharide à la concentration testée ne permet pas la solubilisation de l'insuline. Si la solution est translucide, l'oligosaccharide ou le polysaccharide permet la solubilisation de l'insuline à la concentration testée. De cette façon, la concentration en oligosaccharide ou polysaccharide nécessaire pour solubiliser l'insuline à son point isoélectrique peut être déterminée. Plus cette concentration est basse plus l'affinité de l'oligosaccharide ou du polysaccharide pour l'insuline est importante,

| **Polysaccharide/ Oligosaccharide** | **Solubilisation de l'insuline humaine à 100 UI/mL par le polysaccharide/olig osaccharide à 4mg/mL** | **Solubilisation de l'insuline humaine à 100 UI/mL par le polysaccharide/oligos accharide à 15mg/mL** | **Solubilisation de l'insuline humaine à 100 UI/mL par le polysaccharide/oligo saccharide à 50mg/mL** |
|---|---|---|---|
| **Contre exemples** | | | |
| Polysaccharide 1 | Oui | Oui | Oui |
| Polysaccharide 4 | Oui | Oui | Oui |
| Polysaccharide 3 | Oui | Oui | Oui |
| Polysaccharide 2 | Oui | Oui | Oui |
| Polysaccharide 5 | Oui | Oui | Oui |

| **Exemples** | | | |
|---|---|---|---|
| Oligosaccharide 3 | Non | Oui | Oui |
| Oligosaccharide 6 | Non | Oui | Oui |
| Oligosaccharide 2 | Non | Oui | Oui |
| Oligosaccharide 1 | Non | Non | Non |
| Oligosaccharide 4 | Non | Non | Non |

### E2. Solubilisation de l'insuline lispro à son point isoélectrique

L'insuline lispro a un point isoélectrique à 5,3. A ce pH l'insuline lispro précipite. Un test démontrant la formation d'un complexe de l'insuline lispro avec les différents oligosaccharides ou polysaccharides est exécuté au point isoélectrique. Si une interaction existe, il est possible de solubiliser l'insuline à son point isoélectrique.

La formulation commerciale de l'insuline lispro (Humalog^{®}) est dialysée contre du tampon PO4 1 mM (pH 7). Après dialyse, la concentration en insuline lispro est d'environ 90 UI/mL. Le lyophilisat d'oligosaccharide ou de polysaccharide est pesé et solubilisé dans la solution d'insuline lispro pour mener à des formulations contenant l'insuline lispro à 90 UI/mL et l'oligosaccharide ou le polysaccharide aux concentrations désirés (4, 15 ou 50 mg/mL). Le pH des différentes solutions est ajusté à pH 5,3 par ajout d'acide acétique 200 mM.

L'aspect de la solution est documenté. Si la solution est turbide, l'oligosaccharide ou le polysaccharide à la concentration testée ne permet pas la solubilisation de l'insuline. Si la solution est translucide, l'oligosaccharide ou le polysaccharide permet la solubilisation de l'insuline à la concentration testée. De cette façon, la concentration en oligosaccharide ou en polysaccharide nécessaire pour solubiliser l'insuline à son point isoélectrique peut être déterminée. Plus cette concentration est basse plus l'affinité de l'oligosaccharide ou du polysaccharide pour l'insuline est importante.

| **Polysaccharide/ Oligosaccharide** | **Solubilisation de l'insuline lispro à 90UI/mL par le polysaccharide/oligo saccharide à 4mg/mL** | **Solubilisation de l'insuline lispro à 90UI/mL par le polysaccharide/oligosac charide à 15mg/mL** | **Solubilisation de l'insuline lispro à 90UI/mL par le polysaccharide/oligosac charide à 50mg/mL** |
|---|---|---|---|
| **Contre exemples** | | | |
| Polysaccharide 1 | Oui | Oui | Oui |
| Polysaccharide 3 | Oui | Oui | Oui |
| Polysaccharide 2 | Oui | Oui | Oui |

| **Exemples** | | | |
|---|---|---|---|
| Oligosaccharide 3 | Non | Oui | Oui |
| Oligosaccharide 6 | Non | Oui | Oui |
| Oligosacharide 2 | Non | Non | Oui |
| Oligosaccharide 1 | Non | Non | Non |
| Oligosaccharide 4 | Non | Non | Non |

### F Interaction avec l'albumine

**F1:** Afin de déterminer les interactions entre les différents polysaccharides ou oligosaccharides et une protéine modèle telle que l'albumine, un essai en Centricon (membrane de CutOff 50kD) a été effectué. Une solution de polysaccharide ou d'oligosaccharide à 7,3 mg/mL a été diluée au tiers dans une solution de BSA à 20 mg/mL dans le PBS (concentration dans le mélange : 2,43 mg/mL de polymère, 13,3 mg/mL d'albumine et environ 100mM de sel).

Ce mélange a été centrifugé sur Centricon pour faire passer environ la moitié du volume à travers la membrane. L'albumine est retenue de façon quantitative sur la membrane du Centricon. Les polysaccharides et oligosaccharides analysés passent en grande partie à travers la membrane (pour les polysaccharides ayant des masses molaires les plus importantes, environ 20% du polysaccharide est retenu),

Après centrifugation le polysaccharide ou oligosaccharide est dosé par UV dans le filtrat. Le pourcentage de BC lié à l'albumine est calculé par l'équation suivante :

[polysaccharide ou oligosaccharide dans le filtrat en présence d'albumine]/[ polysaccharide ou oligosaccharide dans le filtrat en absence d'albumine]^{∗}100

On observe très clairement que les polysaccharides de masse molaire 5-15kD sont fortement retenus par l'albumine dans cet essai. Au contraire, les oligosaccharides de plus faible masse molaire 1-2kD sont nettement moins retenus par l'albumine dans cet essai.

| **Polysaccharides/Oligosaccharides** | **%Polysaccharide/Oligosaccharide lié à la BSA** |
|---|---|
| **Contre exemples** | |
| Polysaccharide 4 | 97% |
| Polysaccharide 1 | 95% |
| Polysaccharide 3 | 77% |
| Polysaccharide 5 | 86% |
| Polysaccharide 2 | 82% |

| **Exemples** | |
|---|---|
| Oligosaccharide 4 | 27% |
| Oligosaccharide 1 | 34% |
| Oligosaccharide 2 | 48% |
| Oligosaccharide 3 | 45% |
| Oligosaccharide 6 | 51% |

## Revendications

1. Composition en solution aqueuse, comprenant de l'insuline et au moins un oligosaccharide dont le degré de polymérisation moyen est compris entre 3 et 6 et l'indice de polydispersité Ip est compris entre 1,1 et 2,0, ledit oligosaccharide comportant des groupes fonctionnels carboxyles partiellement substitués, les groupes fonctionnels carboxyles non substitués étant salifiables, et comprenant en outre au moins un composé polyanionique.

2. Composition selon la revendication 1, **caractérisée en ce que** l'insuline est une insuline humaine.

3. Composition selon la revendication 1, **caractérisée en ce que** l'insuline est une insuline analogue.

4. Composition selon l'une quelconque des revendications précédentes, **caractérisée en ce que** le ratio massique oligosaccharide/insuline est compris entre 0,4 et 10.

5. Composition selon l'une quelconque des revendications précédentes, **caractérisée en ce que** la concentration en composé polyanionique est comprise entre 1,4 et 35 mg/mL.

6. Composition selon l'une quelconque des revendications précédentes **caractérisée en ce que** l'oligosaccharide est choisi parmi les oligosaccharides de formule générale I suivante : 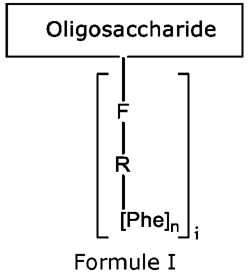 Dans laquelle :
• l'oligosaccharide est un dextrane,
• F résulte du couplage entre le bras de liaison R et une fonction -OH de l'oligosaccharide et étant soit une fonction ester, carbamate ou éther,
• R est une chaîne comprenant entre 1 et 15 carbones, éventuellement branchée et/ou insaturée, comprenant un ou plusieurs hétéroatomes, tels que O, N ou/et S, et ayant au moins une fonction carboxyle,
• Phe est un reste d'un dérivé de phénylalanine, de configuration absolue L ou D, produit du couplage entre l'amine du dérivé de la phénylalanine et au moins un acide porté par le groupement R avant rattachement à Phe,
• n représente la fraction molaire des R substitués par Phe et est comprise entre 0,3 et 0,9, de préférence entre 0,4 et 0,8, encore de préférence entre 0,4 et 0,6,
• i représente la fraction molaire moyenne des groupements F-R-[Phe]ₙ portés par unité saccharidique et est comprise entre 0,5 et 2,5, de préférence entre 0,8 et 1,6, de préférence entre 1,0 et 1,4, de préférence entre 1,0 et 1,2 ;
• - lorsque R n'est pas substitué par Phe, alors le ou les acides du groupement R sont des carboxylates de cation, alcalin de préférence comme Na⁺, K⁺, Ca²⁺ ou Mg²⁺.

7. Composition selon l'une quelconque des revendications 1 à 6, **caractérisée en ce que** le composé polyanionique est choisi dans le groupe constitué des molécules anioniques, des polymères anioniques et des composés constitués d'un squelette formé d'un nombre discret p compris entre 1 et 8 (1 ≤ p ≤ 8) d'unités saccharidiques identiques ou différentes, liées par des liaisons glycosidiques identiques ou différentes naturellement porteurs de groupes carboxyles ou substitués par des groupes carboxyles.

8. Formulation pharmaceutique comprenant une composition selon l'une quelconque des revendications précédentes.

9. Utilisation d'au moins un oligosaccharide dont le degré de polymérisation moyen est compris entre 3 et 6 et l'indice de polydispersité Ip est compris entre 1,1 et 2,0, avec un composé polyanionique, pour préparer une formulation pharmaceutique d'insuline permettant, après administration, d'accélerer le passage de l'insuline dans le sang et de réduire plus rapidement la glycémie par rapport à une formulation exempte d'oligosaccharide seul ou en mélange avec un composé polyanionique.

10. Utilisation selon la revendication 9, **caractérisée en ce que** l'oligosaccharide est choisi parmi les oligosaccharides de formule générale I suivante : 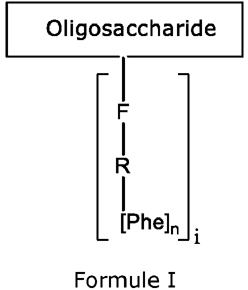 dans laquelle :
• l'oligosaccharide est un dextrane,
• F résulte du couplage entre le bras de liaison R et une fonction -OH de l'oligosaccharide et étant soit une fonction ester, carbamate ou éther,
• R est une chaîne comprenant entre 1 et 15 carbones, éventuellement branchée et/ou insaturée, comprenant un ou plusieurs hétéroatomes, tels que O, N ou/et S, et ayant au moins une fonction carboxyle,
• Phe est un reste d'un dérivé de phénylalanine, de configuration absolue L ou D, produit du couplage entre l'amine du dérivé de la phénylalanine et au moins un acide porté par le groupement R avant rattachement à Phe,
• n représente la fraction molaire des R substitués par Phe et est comprise entre 0,3 et 0,9, de préférence entre 0,4 et 0,8, encore de préférence entre 0,4 et 0,6,
• i représente la fraction molaire moyenne des groupements F-R-[Phe]ₙ portés par unité saccharidique et est comprise entre 0,5 et 2,5, de préférence entre 0,8 et 1,6, de préférence entre 1,0 et 1,4, de préférence entre 1,0 et 1,2 ;
• - lorsque R n'est pas substitué par Phe, alors le ou les acides du groupement R sont des carboxylates de cation, alcalin de préférence comme Na⁺ ou K⁺.

11. Utilisation selon l'une des revendications 9 ou 10, **caractérisée en ce que** le composé polyanionique est choisi dans le groupe constitué des molécules anioniques, des polymères anioniques et des composés constitués d'un squelette formé d'un nombre discret p compris entre 1 et 8 (1 ≤ p ≤ 8) d'unités saccharidiques identiques ou différentes, liées par des liaisons glycosidiques identiques ou différentes naturellement porteurs de groupe carboxyles ou substitués par des groupes carboxyles.

12. Méthode de préparation d'une formulation d'insuline humaine ayant une concentration en insuline comprise entre 240 et 3000 µM (40 et 500 UI/mL), dont le délai d'action chez l'humain est inférieur à celui de la formulation de référence à la même concentration en insuline en l'absence d'oligosaccharide, **caractérisée en ce qu'**elle comprend une étape d'addition à ladite formulation d'au moins un oligosaccharide dont le degré de polymérisation moyen est compris entre 3 et 6 et l'indice de polydispersité Ip est est compris entre 1,1 et 2,0 comportant des groupes fonctionnels carboxyles partiellement substitués, et comprenant en outre une étape d'addition à ladite formulation d'au moins un composé polyanionique.

13. Méthode de préparation d'une formulation d'insuline analogue ayant une concentration en insuline comprise entre 240 et 3000 µM (40 et 500 UI/mL), dont le délai d'action chez l'humain est inférieur à celui de la formulation de référence à la même concentration en insuline en l'absence d'oligosaccharide, **caractérisée en ce qu'**elle comprend une étape d'addition à ladite formulation d'au moins un oligosaccharide dont le degré de polymérisation moyen est compris entre 3 et 6 et l'indice de polydispersité Ip est est compris entre 1,1 et 2,0 comportant des groupes fonctionnels carboxyles partiellement substitués, et comprenant en outre une étape d'addition à ladite formulation d'au moins un composé polyanionique.

14. Méthode selon la revendication 12 ou 13 **caractérisée en ce que** que l'oligosaccharide est choisi parmi les oligosaccharides de formule générale I suivante : 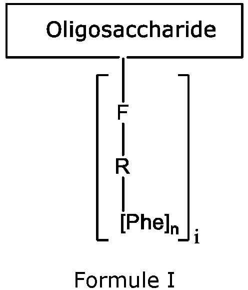 dans laquelle :
• l'oligosaccharide est un dextrane,
• F résulte du couplage entre le bras de liaison R et une fonction -OH de l'oligosaccharide et étant soit une fonction ester, carbamate ou éther,
• R est une chaîne comprenant entre 1 et 15 carbones, éventuellement branchée et/ou insaturée, comprenant un ou plusieurs hétéroatomes, tels que O, N ou/et S, et ayant au moins une fonction carboxyle,
• Phe est un reste d'un dérivé de phénylalanine, de configuration absolue L ou D, produit du couplage entre l'amine du dérivé de la phénylalanine et au moins un acide porté par le groupement R avant rattachement à Phe,
• n représente la fraction molaire des R substitués par Phe et est comprise entre 0,3 et 0,9, de préférence entre 0,4 et 0,8, encore de préférence entre 0,4 et 0,6,
• i représente la fraction molaire moyenne des groupements F-R-[Phe]ₙ portés par unité saccharidique et est comprise entre 0,5 et 2,5, de préférence entre 0,8 et 1,6, de préférence entre 1,0 et 1,4, de préférence entre 1,0 et 1,2 ;
• - lorsque R n'est pas substitué par Phe, alors le ou les acides du groupement R sont des carboxylates de cation, alcalin de préférence comme Na⁺ ou K⁺.

15. Méthode selon l'une des revendications 12 ou 13 **caractérisée en ce que** le composé polyanionique est choisi dans le groupe constitué des molécules anioniques, des polymères anioniques et des composés constitués d'un squelette formé d'un nombre discret p compris entre 1 et 8 (1 ≤ p ≤ 8) d'unités saccharidiques identiques ou différentes, liées par des liaisons glycosidiques identiques ou différentes naturellement porteurs de groupe carboxyles ou substitués par des groupes carboxyles.

## Patentansprüche

1. Zusammensetzung in wässriger Lösung, umfassend Insulin und mindestens ein Oligosaccharid, dessen durchschnittlicher Polymerisationsgrad zwischen 3 und 6 liegt und dessen Polydispersitätsindex Ip zwischen 1,1 und 2,0 liegt, wobei das Oligosaccharid teilweise substituierten funktionellen Carboxylgruppen aufweist, wobei die unsubstituierten funktionellen Carboxylgruppen salzbildend sind, und ferner umfassend mindestens eine polyanionische Verbindung.

2. Zusammensetzung nach Anspruch 1, **dadurch gekennzeichnet, dass** das Insulin Humaninsulin ist.

3. Zusammensetzung nach Anspruch 1, **dadurch gekennzeichnet, dass** das Insulin ein Analoginsulin ist.

4. Zusammensetzung nach einem der vorhergehenden Ansprüche, **dadurch gekennzeichnet, dass** das Massenverhältnis Oligosaccharid/Insulin zwischen 0,4 und 10 liegt.

5. Zusammensetzung nach einem der vorhergehenden Ansprüche, **dadurch gekennzeichnet, dass** die Konzentration der polyanionischen Verbindung zwischen 1,4 und 35 mg/mL liegt.

6. Zusammensetzung nach einem der vorhergehenden Ansprüche, **dadurch gekennzeichnet, dass** das Oligosaccharid unter den Oligosacchariden der nachstehenden allgemeinen Formel I ausgewählt ist: 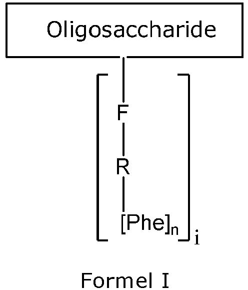 worin:
• das Oligosaccharid ein Dextran ist,
• F aus der Kopplung zwischen dem Linker R und einer -OH-Funktion des Oligosaccharids entsteht und entweder eine Ester-, Carbamat- oder Etherfunktion ist,
• R eine Kette ist, die zwischen 1 und 15 Kohlenstoffatome umfasst, gegebenenfalls verzweigt und/oder ungesättigt ist, die ein oder mehrere Heteroatome wie O, N und/oder S umfasst und mindestens eine Carboxylfunktion aufweist,
• Phe ist ein Rest eines Phenylalaninderivats mit der absoluten Konfiguration L oder D, Produkt der Kopplung zwischen dem Amin des Phenylalaninderivats und mindestens einer Säure, die von der R-Gruppe vor der Bindung an Phe getragen wird,
• n stellt den molaren Anteil der durch Phe substituierten R dar und liegt zwischen 0,3 und 0,9, vorzugsweise zwischen 0,4 und 0,8, besonders bevorzugt zwischen 0,4 und 0,6,
• i stellt den durchschnittlichen molaren Anteil der pro Saccharideinheit getragenen F-R-[Phe]ₙ-Gruppen dar und liegt zwischen 0,5 und 2,5, vorzugsweise zwischen 0,8 und 1,6, vorzugsweise zwischen 1,0 und 1,4, vorzugsweise zwischen 1,0 und 1,2 ;
• - wenn R nicht durch Phe substituiert ist, dann sind die Säure oder Säuren der R-Gruppe Kationencarboxylate, vorzugsweise alkalisch wie Na⁺, K⁺, Ca²⁺ oder Mg²⁺.

7. Zusammensetzung nach einem der vorhergehenden Ansprüche 1 bis 6, **dadurch gekennzeichnet, dass** die polyanionische Verbindung aus der Gruppe ausgewählt ist, die aus anionischen Molekülen, anionischen Polymeren und Verbindungen besteht, die aus einem Gerüst bestehen, das aus einer diskreten Zahl p zwischen 1 und 8 (1 ≤ p ≤ 8) aus gleichen oder verschiedenen Saccharideinheiten gebildet ist, die durch gleiche oder verschiedene glykosidische Bindungen verknüpft sind, die natürlicherweise Carboxylgruppen tragen oder durch Carboxylgruppen substituiert sind.

8. Pharmazeutische Formulierung, umfassend eine Zusammensetzung nach einem der vorhergehenden Ansprüche.

9. Verwendung mindestens eines Oligosaccharids mit einem durchschnittlichen Polymerisationsgrad zwischen 3 und 6 und einem Polydispersitätsindex Ip zwischen 1,1 und 2,0 mit einer polyanionischen Verbindung zur Herstellung einer pharmazeutischen Insulinformulierung, die nach der Verabreichung, den Übergang von Insulin in den Blutkreislauf beschleunigt und die Glykämie im Vergleich zu einer oligosaccharidfreien Formulierung allein oder in Mischung mit einer polyanionischen Verbindung schneller senkt.

10. Verwendung nach Anspruch 9, **dadurch gekennzeichnet, dass** das Oligosaccharid unter den Oligosacchariden der nachstehenden allgemeinen Formel I ausgewählt ist: 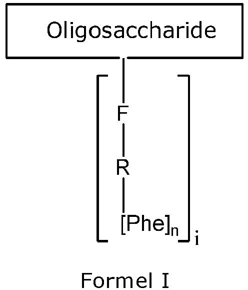 worin:
• das Oligosaccharid ein Dextran ist,
• F aus der Kopplung zwischen dem Linker R und einer -OH-Funktion des Oligosaccharids entsteht und entweder eine Ester-, Carbamat- oder Etherfunktion ist,
• R eine Kette ist, die zwischen 1 und 15 Kohlenstoffatome umfasst, gegebenenfalls verzweigt und/oder ungesättigt ist, die ein oder mehrere Heteroatome wie O, N und/oder S umfasst und mindestens eine Carboxylfunktion aufweist,
• Phe ist ein Rest eines Phenylalaninderivats mit der absoluten Konfiguration L oder D, Produkt der Kopplung zwischen dem Amin des Phenylalaninderivats und mindestens einer Säure, die von der R-Gruppe vor der Bindung an Phe getragen wird,
• n stellt den molaren Anteil der durch Phe substituierten R dar und liegt zwischen 0,3 und 0,9, vorzugsweise zwischen 0,4 und 0,8, besonders bevorzugt zwischen 0,4 und 0,6,
• i stellt den durchschnittlichen molaren Anteil der pro Saccharideinheit getragenen F-R-[Phe]ₙ-Gruppen dar und liegt zwischen 0,5 und 2,5, vorzugsweise zwischen 0,8 und 1,6, vorzugsweise zwischen 1,0 und 1,4, vorzugsweise zwischen 1,0 und 1,2 ;
• - wenn R nicht durch Phe substituiert ist, dann sind die Säure oder Säuren der R-Gruppe Kationencarboxylate, vorzugsweise alkalisch wie Na⁺ und K⁺.

11. Verwendung nach einem der Ansprüche 9 bis 10, **dadurch gekennzeichnet, dass** die polyanionische Verbindung aus der Gruppe ausgewählt ist, die aus anionischen Molekülen, anionischen Polymeren und Verbindungen besteht, die aus einem Gerüst bestehen, das aus einer diskreten Zahl p zwischen 1 und 8 (1 ≤ p ≤ 8) aus gleichen oder verschiedenen Saccharideinheiten gebildet ist, die durch gleiche oder verschiedene glykosidische Bindungen verknüpft sind, die natürlicherweise Carboxylgruppen tragen oder durch Carboxylgruppen substituiert sind.

12. Verfahren zur Herstellung einer Humaninsulinformulierung mit einer Insulinkonzentration zwischen 240 und 3000 µM (40 und 500 UI/mL), deren Wirkungsfrist beim Menschen geringer ist als der der Referenzformulierung bei gleicher Insulinkonzentration in Abwesenheit von Oligosaccharid, **dadurch gekennzeichnet, dass** es einen Schritt der Zugabe mindestens eines Oligosaccharids zu der Formulierung umfasst, dessen durchschnittlicher Polymerisationsgrad zwischen 3 und 6 liegt und dessen Polydispersitätsindex Ip zwischen 1,1 und 2,0 liegt und das teilweise substituierte funktionelle Carboxylgruppen aufweist, und, dass es ferner einen Schritt der Zugabe mindestens einer polyanionischen Verbindung zu der Formulierung umfasst.

13. Verfahren zur Herstellung einer Analoginsulinformulierung mit einer Insulinkonzentration zwischen 240 und 3000 µM (40 und 500 UI/mL), deren Wirkungsfrist beim Menschen geringer ist als der der Referenzformulierung bei gleicher Insulinkonzentration in Abwesenheit von Oligosaccharid, **dadurch gekennzeichnet, dass** es einen Schritt der Zugabe mindestens eines Oligosaccharids zu der Formulierung umfasst, dessen durchschnittlicher Polymerisationsgrad zwischen 3 und 6 liegt und dessen Polydispersitätsindex Ip zwischen 1,1 und 2,0 liegt und das teilweise substituierte funktionelle Carboxylgruppen aufweist, und, dass es ferner einen Schritt der Zugabe mindestens einer polyanionischen Verbindung zu der Formulierung umfasst.

14. Verfahren nach Anspruch 12 oder 13, **dadurch gekennzeichnet, dass** das Oligosaccharid unter den Oligosacchariden der nachstehenden allgemeinen Formel I ausgewählt ist: 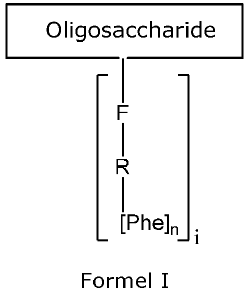 worin:
• das Oligosaccharid ein Dextran ist,
• F aus der Kopplung zwischen dem Linker R und einer -OH-Funktion des Oligosaccharids entsteht und entweder eine Ester-, Carbamat- oder Etherfunktion ist,
• R eine Kette ist, die zwischen 1 und 15 Kohlenstoffatome umfasst, gegebenenfalls verzweigt und/oder ungesättigt ist, die ein oder mehrere Heteroatome wie O, N und/oder S umfasst und mindestens eine Carboxylfunktion aufweist,
• Phe ist ein Rest eines Phenylalaninderivats mit der absoluten Konfiguration L oder D, Produkt der Kopplung zwischen dem Amin des Phenylalaninderivats und mindestens einer Säure, die von der R-Gruppe vor der Bindung an Phe getragen wird,
• n stellt den molaren Anteil der durch Phe substituierten R dar und liegt zwischen 0,3 und 0,9, vorzugsweise zwischen 0,4 und 0,8, besonders bevorzugt zwischen 0,4 und 0,6,
• i stellt den durchschnittlichen molaren Anteil der pro Saccharideinheit getragenen F-R-[Phe]ₙ-Gruppen dar und liegt zwischen 0,5 und 2,5, vorzugsweise zwischen 0,8 und 1,6, vorzugsweise zwischen 1,0 und 1,4, vorzugsweise zwischen 1,0 und 1,2 ;
• - wenn R nicht durch Phe substituiert ist, dann sind die Säure oder Säuren der R-Gruppe Kationencarboxylate, vorzugsweise alkalisch wie Na⁺ und K⁺.

15. Verfahren nach einem der Ansprüche 12 bis 13, **dadurch gekennzeichnet, dass** die polyanionische Verbindung aus der Gruppe ausgewählt ist, die aus anionischen Molekülen, anionischen Polymeren und Verbindungen besteht, die aus einem Gerüst bestehen, das aus einer diskreten Zahl p zwischen 1 und 8 (1 ≤ p ≤ 8) aus gleichen oder verschiedenen Saccharideinheiten gebildet ist, die durch gleiche oder verschiedene glykosidische Bindungen verknüpft sind, die natürlicherweise Carboxylgruppen tragen oder durch Carboxylgruppen substituiert sind.

## Claims

1. A composition in aqueous solution, comprising insulin and at least one oligosaccharide whose average degree of polymerization is between 3 and 13 and whose polydispersity index PDI is comprised from 1.1 to 2.0, said oligosaccharide having partially substituted carboxyl functional groups, the unsubstituted carboxyl functional groups being salifiable and comprising at least one polyanionic compound.

2. The composition as claimed in claim 1, **characterized in that** the insulin is a human insulin.

3. The composition as claimed in claim 1, **characterized in that** the insulin is an insulin analog.

4. The composition as claimed in any one of the preceding claims, **characterized in that** the oligosaccharide/insulin weight ratio is between 0.4 and 10.

5. The composition as claimed in any one of the preceding claims, **characterized in that** the concentration of polyanionic compound is between 1.4 and 35 mg/mL.

6. The composition as claimed in any one of the preceding claims, **characterized in that** the oligosaccharide is selected from the oligosaccharides of the following general formula I: 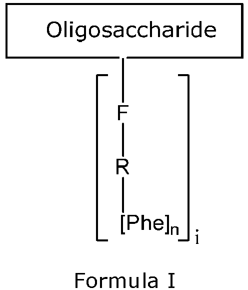 In which:
• the oligosaccharide is a dextran,
• F results from the coupling between linker arm R and an -OH function of the oligosaccharide and being either an ester , carbamate or ether function,
• R is a chain comprising between 1 and 15 carbons, optionally branched and/or unsaturated, comprising one or more heteroatoms, such as O, N and/or S, and having at least one carboxyl function,
• Phe is a residue of a phenylalanine derivative, of absolute configuration L or D, produced from coupling between the amine of the phenylalanine derivative and at least one acid carried by group R prior to attachment to Phe,
• n represents the mole fraction of R substituted with Phe and is between 0.3 and 0.9, preferably between 0.4 and 0.8, more preferably between 0.4 and 0.6,
• i represents the average mole fraction of groups F-R-[Phe]ₙ borne per saccharide unit and is between 0.5 and 2.5, preferably between 0.8 and 1.6, preferably between 1.0 and 1.4, preferably between 1.0 and 1.2;
• -when R is not substituted with Phe, the acid or acids of group R are carboxylates with an alkaline cation, preferably such as Na⁺, K⁺, Ca²⁺ or Mg²⁺.

7. The composition as claimed in any one of claims 2 to 9, **characterized in that** the anionic compound is selected from the group consisting of anionic molecules, anionic polymers and of compounds consisting of a skeleton formed from a discrete number p between 1 and 8 (1 ≤ p ≤ 8) of identical or different saccharide units, bound by identical or different glycosidic bonds that are naturally carriers of carboxyl groups or are substituted with carboxyl groups.

8. A pharmaceutical formulation comprising a composition as claimed in any one of the preceding claims.

9. The use of at least one oligosaccharide whose average degree of polymerization is between 3 and 13 and whose polydispersity index PDI is comprised from 1.1 to 2.0, with a polyanionic compound, for preparing a pharmaceutical formulation of insulin that makes it possible, after administration, to accelerate the passage of insulin into the blood and reduce glycemia more rapidly relative to an oligosaccharide-free formulation alone or mixed with a polyanionic compound.

10. The use as claimed in claim 9, **characterized in that** the oligosaccharide is selected from the oligosaccharides of the following general formula I: 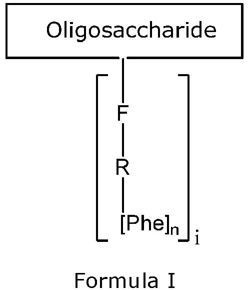 In which:
• the oligosaccharide is a dextran,
• F results from the coupling between linkage R and an -OH function of the oligosaccharide and being either an ester function, carbamate or ether function,
• R is a chain comprising between 1 and 15 carbons, optionally branched and/or unsaturated, comprising one or more heteroatoms, such as O, N and/or S, and having at least one carboxyl function,
• Phe is a residue of a phenylalanine derivative, of absolute configuration L or D, produced fromcoupling between the amine of the phenylalanine derivative and at least one acid carried by group R prior to attachment to Phe,
• n represents the mole fraction of R substituted with Phe and is between 0.3 and 0.9, preferably between 0.4 and 0.8, more preferably between 0.4 and 0.6,
• i represents the average mole fraction of groups F-R-[Phe]ₙ borne per saccharide unit and is between 0.5 and 2.5, preferably between 0.8 and 1.6, preferably between 1.0 and 1.4, preferably between 1.0 and 1.2;
• - when R is not substituted with Phe, the acid or acids of group R are carboxylates with an alkaline cation, preferably such as Na⁺ or K⁺.

11. The use as claimed in one of Claims 9 or 10, **characterized in that** the anionic compound is selected from the group consisting of anionic molecules, anionic polymers and of compounds consisting of a skeleton formed from a discrete number p between 1 and 8 (1 ≤ p ≤ 8) of identical or different saccharide units, bound by identical or different glycosidic bonds that are naturally carriers of carboxyl groups or are substituted with carboxyl groups.

12. A method of preparing a formulation of human insulin having an insulin concentration between 240 and 3000 µM (40 and 500 IU/mL), whose onset of in action in humans is less than that of the reference formulation at the same insulin concentration in the absence of oligosaccharide, **characterized in that** it comprises a step of adding, to said formulation, at least one oligosaccharide whose average degree of polymerization is between 3 and 13 and polydispersity index PDI is comprised from 1.1 to 2.0 having partially substituted carboxyl functional groups and comprising at least one polyanionic compound.

13. A method of preparing a formulation of insulin analog having an insulin concentration between 240 and 3000 µM (40 and 500 IU/mL), whose onset of action in humans is less than that of the reference formulation at the same insulin concentration in the absence of oligosaccharide whose average degree of polymerization is between 3 and 13 and polydispersity index PDI is comprised from 1.1 to 2.0 having partially substituted carboxyl functional groups and comprising at least one polyanionic compound.

14. The method as claimed in any one of claims 12 to 13, **characterized in that** the oligosaccharide is selected from the oligosaccharides of the following general formula I: 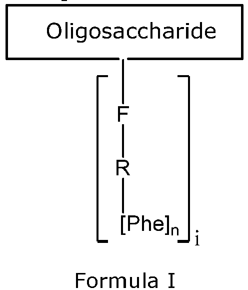 In which:
• the oligosaccharide is a dextran,
• F results from the coupling between linkage R and an -OH function of the oligosaccharide and being either an ester- carbamate or ether function,
• R is a chain comprising between 1 and 15 carbons, optionally branched and/or unsaturated, comprising one or more heteroatoms, such as O, N and/or S, and having at least one carboxyl function,
• Phe is a residue of a phenylalanine derivative, of absolute configuration L or D, produced from coupling between the amine of the phenylalanine derivative and at least one acid carried by group R prior to attachment to Phe,
• n represents the mole fraction of R substituted with Phe and is between 0.3 and 0.9, preferably between 0.4 and 0.8, more preferably between 0.4 and 0.6,
• i represents the average mole fraction of groups F-R-[Phe]ₙ borne per saccharide unit and is between 0.5 and 2.5, preferably between 0.8 and 1.6, preferably between 1.0 and 1.4, preferably between 1.0 and 1.2;
• - when R is not substituted with Phe, the acid or acids of group R are carboxylates with an alkaline cation, preferably such as Na⁺ or K⁺.

15. The method as claimed in one of Claims 12 or 13, **characterized in that** the anionic compound is selected from the group consisting of anionic molecules, anionic polymers and of compounds consisting of a skeleton formed from a discrete number p between 1 and 8 (1 ≤ p ≤ 8) of identical or different saccharide units, bound by identical or different glycosidic bonds that are naturally carriers of carboxyl groups or are substituted with carboxyl groups.
